(19) Europäisches Patentamt

European Patent Office

Office européen des brevets

(11) **EP 1 707 213 A1**

(12) **EUROPÄISCHE PATENTANMELDUNG**

(43) Veröffentlichungstag:
04.10.2006 Patentblatt 2006/40

(51) Int Cl.:
*A61K 36/537* (2006.01)        *A61K 36/28* (2006.01)
*A61P 17/02* (2006.01)

(21) Anmeldenummer: 05005868.4

(22) Anmeldetag: 17.03.2005

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(71) Anmelder: **Kern, Elisabeth**
**4040 Linz (AT)**

(72) Erfinder:
• **Kern, Elisabeth**
**4040 Linz (AT)**

• **Kopp, Brigitte**
**1180 Wien (AT)**
• **Locker, Gottfried**
**1030 Wien (AT)**
• **Schmidinger, Manuela**
**1080 Wien (AT)**
• **Zielinski, Christoph**
**1090 Wien (AT)**

(74) Vertreter: **Kador & Partner**
**Corneliusstrasse 15**
**80469 München (DE)**

(54) **Pflanzliche Zusammensetzung zur Behandlung von erythematösen Hautveränderungen**

(57)    Die Erfindung bezieht sich auf eine pflanzliche Zusammensetzung, aus ihr hergestellte Extrakte, Tinkturen, Salben, Cremes, Emulsionen oder Gele sowie deren Verwendung zur Herstellung eines Arzneimittels zur Behandlung von erythematösen Hautveränderungen.

Die Zusammensetzung umfaßt Calendula officinalis L. enthaltend Calendulae flos, Matricaria recutita L. enthaltend Matricariae flos und Salvia officinalis L. enthaltend Salviae officinalis folium.

EP 1 707 213 A1

**Beschreibung**

Die Erfindung bezieht sich auf eine pflanzliche Zusammensetzung, aus ihr hergestellte Extrakte, Tinkturen, Salben, Cremes, Emulsionen oder Gele sowie deren Verwendung zur Herstellung eines Arzneimittels zur Behandlung von erythematösen Hautveränderungen.

In der Vergangenheit konnte durch umfangreiche Studien belegt werden, dass zahlreiche pflanzliche Mischungen pharmazeutische Wirkung besitzen.

Auch in der Krebstherapie werden zur effektiveren Behandlung sogenannte Phytopharmaka eingesetzt. Dabei stellen sie jedoch keine Alternativmethoden dar, sondern dienen vielmehr als begleitende Heilmethoden zur konventionellen Behandlung mit Zytostatika.

Im Folgenden werden die Begriffe Zytostatikum und Chemotherapeutikum, sowie Zytostatikatherapie und Chemotherapie äquivalent verwendet.

Die Anwendungsbereiche der Phytopharmaka in der Chemotherapie sind vielfältig. So werden zum Beispiel Enzian- oder Wermutkraut aufgrund ihrer Bitterstoffe gegen die bei der Chemotherapie auftretenden Magen- und Darmbeschwerden eingesetzt. Ingwer hilft bei Übelkeit und Erbrechen.

Ein Hauptproblem der bei einer Chemotherapie auftretenden Nebenwirkungen ist nicht nur, dass es sich um für den Patienten unangenehme Begleiterscheinungen handelt, sondern, dass sie bei entsprechendem Ausmaß den Abbruch der Zytostatikatherapie erzwingen.

So wurde beobachtet, dass durch den Einsatz des Zytostatikums 5-Fluorouracil - im Folgenden 5-FU genannt - oder $N_4$-Pentyloxycarbonyl-5'desoxy-Fluorocytidin - im Folgenden Capecitabin genannt - bei 50% der Patienten als häufigste Nebenwirkung eine palmoplantare Erythrodysästhesie, auch als erythematöse Hautveränderung oder Hand-Fuß-Syndrom bezeichnet, auftrat, wie sie ebenfalls bei der Sichelzellenanämie zu beobachten ist. Dabei bilden sich schmerzhafte Hautveränderungen und es kommt zur Blasenbildung im Bereich der Handinnenflächen und an den Fußsohlen (akraler Bereich). Abhängig vom Schweregrad und dem Ausmaß der Hautläsion ist der betroffene Patient leicht bis schwer in seinem täglichen Leben beeinträchtigt. Mitunter ist es dem Patienten aufgrund der Entzündung an den Fußflächen nicht einmal mehr möglich zu gehen. Das Auftreten der erythematösen Hautveränderung erfordert in den meisten Fällen das Pausieren der Chemotherapie, allenfalls eine Dosisreduzierung, was in beiden Fällen die Therapieeffektivität erheblich herabsetzt. Ein Schweregrad von 3 oder 4 der erythematösen Hautveränderung kann sogar zum Abbruch der Behandlung führen.

Die Schweregrade werden laut Weltgesundheitsorganisation (WHO; Handbook for reporting of cancer treatment, *No. 48*, **1979,** WHO Offset Publications, Geneva) wie folgt unterteilt:

Grad 0: Keine auffälligen Hautreaktionen.

Grad 1: Taubheitsgefühl, Dysästhesie/ Parästhesie, Kribbeln, schmerzlose Schwellungen oder Erythem der Hände und/oder Füße und/oder Beschwerden, die den Alltag nicht beeinträchtigen.

Grad 2: Trockene Schuppung, Blasen, Juckreiz, schmerzhaftes Erythem und Schwellung der Hände und/oder Füße und/oder Beschwerden, die die Aktivitäten des Patienten im täglichen Leben beeinträchtigen.

Grad 3: Feuchte Abschuppungen, Geschwürbildung, Blasenbildung und starke Schmerzen an den Händen und/ oder Füßen und/oder starke Beschwerden, die es für den Patienten unmöglich machen zu arbeiten oder Aktivitäten des täglichen Lebens auszuführen.

Grad 4: Exfoliative dermatitis, Nekrosen, chirurgische Therapie erforderlich.

Nach Abklingen der Symptome ist zwar eine Wiederaufnahme der Zytostatikumtherapie möglich, jedoch teilweise mit herabgesetzter Dosierung. Somit kann eine Unterbrechung oder Dosisminimierung der Chemotherapie nicht als zielgerichtete Behandlung des Hand-Fuß-Syndroms angesehen werden.

Die genaue Pathogenese der chemoinduzierten erythematösen Hautveränderung ist unbekannt, daher sind die Therapiemöglichkeiten relativ eingeschränkt. Die Behandlungen orientieren sich vor allem an der Ätiologie der Erkrankungen und greifen zumeist auf eine symptomatische topische Therapie zurück.

Bei einer Chemotherapie mit 5-FU oder seinen Derivaten vermutet man, dass durch das Auftreten von einem oder mehreren 5-FU-Kataboliten bei der Behandlung die erythematöse Hautveränderung ausgelöst wird. Es konnte beobachtet werden, dass bei Gabe von Dehydropyrimidin-Dehydrogenase -Inhibitoren (DPD-Inhibitoren) das Hand-Fuß-Syndrom bei den Patienten gelindert werden kann, was die wesentliche Rolle der 5-FU-Kataboliten bei der Entstehung des Syndroms unterstreicht, jedoch aufgrund der zahlreichen ungeklärten Einzelheiten der Pathogenese bei erythematösen Hautveränderungen geht man auch von anderen Entstehungsmechanismen aus.

So beschreiben Lopez A.M. et al. in Cancer Chemother. Pharmacol., 1999, 44, 303-306 eine weitere Therapiemöglichkeit. Um der auftretenden erythematösen Hautveränderung entgegenzuwirken, behandelten sie die Patienten mit 99%igem Dimethylsulfoxid (DMSO) während der intravenösen Gabe von Doxorubicin.

Coleman et al. berichten auf der Tagung der American Society of Clinical Oncology, ASCO's 2001, San Francisco, 12. - 15. Mai, über die Möglichkeit einer supportiven Behandlung eines durch Doxil induzierten Hand-Fuß-Syndroms. Durch die Gabe von Dexamethason war es möglich, die Chemotherapie mit der ursprünglichen Dosierung des Doxorubicinderivates Doxil fortzuführen, wenngleich die behandelten Patienten unter diesem Zytostatikum ein Hand-Fuß-Syndrom vom Grade 2 bis 4 ausgebildet hatten. Daraus resultierend schloss man, dass durch eine interventive Gabe des Glucocorticoids diese dermatologische Toxizität verhindert bzw. wieder beseitigt werden kann.

Den in der Literatur beschriebenen Medikamenten zur Behandlung von chemoinduzierten erythematösen Hautveränderungen ist jedoch zu eigen, dass sie entweder nur ein durch ein bestimmtes Chemotherapeutikum ausgelöstes Hand-Fuß-Syndrom behandeln können, wie zum Beispiel bei der Gabe von DPD-Inhibitoren, oder dass sie selber erhebliche Nebenwirkungen aufweisen, wie zum Beispiel die Corticosteroide oder DMSO. Des weiteren ist die Bereitschaft (Compliance) der Patienten, solche Medikamente zusätzlich zur Zytostatikatherapie zu verwenden, eingeschränkt.

Ziel der vorliegenden Erfindung ist es daher eine Zusammensetzung zur Verfügung zu stellen, die zum einen eine schnelle Linderung bzw. Heilung des Hand-Fuß-Syndroms herbeiführt, zum anderen es ermöglicht, die Dosierung mit dem entsprechenden Chemotherapeutikum während der Therapie beizubehalten, keine Nebenwirkungen hervorruft, die Behandlung mit der Zusammensetzung bzw. deren Anwendung für die Patienten einfach ist, damit deren Compliance, die Zusammensetzung anzuwenden, steigt.

Aus diesem Grund stellt die vorliegende Erfindung eine pflanzliche Zusammensetzung zur Verfügung, die sich zur Herstellung eines Arzneimittels für die Behandlung von erythematösen Hautveränderungen eignet und aufgrund der Zusammensetzung und Anwendungsart eine hohe Akzeptanz bei den Patienten findet.

Wie im Folgenden offenbart, eignet sich eine Zusammensetzung umfassend Calendula officinalis L. (Ringelblume) enthaltend Calendulae flos, Matricaria recutita L. (Echte Kamille) enthaltend Matricariae flos und Salvia officinalis L. (Echter Salbei) enthaltend Salviae officinalis folium zur Behandlung von erythematösen Hautverärlderungen besonders gut.

Dabei ist vorzugsweise zu beachten, dass die Drogenbestandteile mit einem Mindestgehalt in der Zusammensetzung vorkommen und von guter pharmazeutischer Qualität sind.

Des weiteren zeigte sich, dass aus der erfindungsgemäßen Zusammensetzung hergestellte Extrakte, Tinkturen, Salben, Cremes, Emulsionen oder Gele sich ebenfalls zur Behandlung von erythematösen Hautveränderungen eignen.

Die vorliegende Erfindung stellt demnach eine Zusammensetzung umfassend Calendula officinalis L. enthaltend Calendulae flos , Matricaria recutita L. enthaltend Matricariae flos und Salvia officinalis L. enthaltend Salviae officinalis folium zur Verfügung.

Ebenso lassen sich Extrakte, Tinkturen, Salben, Cremes, Emulsionen sowie Gele aus der Zusammensetzung herstellen.

Die Zusammensetzung bzw. deren Extrakte, Tinkturen, Salben, Cremes, Emulsionen oder Gele können zur Herstellung eines Arzneimittels zur Behandlung von erythematösen Hautveränderungen verwendet werden, im Speziellen zur Behandlung der akralen erythematösen Hautveränderung, ohne dass die Zytostatikatherapie unterbrochen werden muss oder es zu einer Dosisminimierung des eingesetzten Chemotherapeutikums kommt. Des weiteren ruft die Behandlung mit der erfindungsgemäßen Zusammensetzung keine Nebenwirkungen hervor und findet eine positive Compliance bei den Patienten.

Grundlage der Erfindung ist eine Zusammensetzung, die Calendula officinalis L. (Ringelblume), Matricaria recutita L. (Echte Kamille; alte Bezeichnung: Chamomilla recutita (L.).) und Salvia officinalis L. (Echter Salbei), umfasst.

Diese Pflanzen zeichnen sich unter anderem dadurch aus, dass sie die Arzneidrogen Calendulae flos, Matricariae flos und Salviae officinalis folium enthalten.

Bei Calendulae flos handelt es sich um die Blüten der Ringelblume, bestehend aus den ganzen oder geschnittenen, entfalteten, getrockneten und vom Blütenstandboden befreiten Einzelblüten der kultivierten, gefüllten Varietät von Calendula officinalis L..

Unter Matricariae flos versteht man die getrockneten Blütenköpfchen von Matricaria recutita L..

Salviae officinalis folium sind ganze oder geschnittene Laubblätter von Salvia officinalis L..

Diesen Arzneidrogen ist vor allem die antiphlogistische Wirkung gemein. Ebenso weist jede für sich granulationsfördernde, wundheilungsfördernde und antibakterielle Effekte auf.

Die Anwendung jeder einzelnen Arzneidroge für sich bei verschiedenen Indikationen ist aus der Literatur bekannt. So werden Ringelblumensalben zur Entzündungshemmung und wundheilungsfördernden Therapie verwendet (Hänsel R. et. al., 1999, Pharmakognosie - Phytopharmazie, 7. Aufl., Springer Verlag, 524 - 526, 699 - 704, 740 - 743).

Studien belegen, dass Kamille als Antiphlogistikum eingesetzt werden kann (Kamillosan® im Spiegel der Literatur (1991), Hrsg. Th. Neseman, R. Patzelt-Wenczler, Frankfurt am Main; pmi Verl., 24 - 27).

Salbei weist antiinflammatorische Eigenschaften auf (Baricevic C.H., 1991, Zeitschrift für Phytotherapie, 12, 61 - 69).

Die erfindungsgemäße Kombination der drei Arzneidrogen führt zu einem synergistischen Effekt. Synergistischer Effekt heißt, dass die Wirkung einer Arzneidroge durch die Kombination mit einer oder mehreren Arzneidrogen eine Wirkung aufweist, die stärker ist, als die Summe der Einzelwirkungen der einzelnen Arzneidrogen für sich.

Um jedoch den gewünschten Behandlungserfolg bei der erythematösen Hauterkrankung mit der erfindungsgemäßen Zusammensetzung, deren Extrakte, Tinkturen, Salben, Cremes, Emulsionen oder Gele zu erhalten, zeigte es sich, wie bereits erwähnt, dass der Heilungserfolg davon abhängig ist, wie hoch der Anteil der Arzneidrogen Calendulae flos, Matricariae flos, Salviae officinalis folium in der Zusammensetzung ist.

Daher ist eine Zusammensetzung mit einem Anteil an Calendulae flos von mindestens 40 Gew. % bezogen auf die Gesamtmenge Calendula officinalis L., einem Anteil an Matricariae flos von mindestens 40 Gew. % bezogen auf die Gesamtmenge Matricaria recutita L. und einem Anteil an Salviae officinalis folium von mindestens 40 Gew. % bezogen auf die Gesamtmenge Salvia officinalis L. bevorzugt.

Besonders bevorzugt ist eine Zusammensetzung, die einen Anteil von Calendulae flos von 40 bis 60 Gew. % bezogen auf die Gesamtmenge Calendula officinalis L., von Matricariae flos von 40 bis 60 Gew. % bezogen auf die Gesamtmenge Matricaria recutita L. und von Salviae officinalis folium von 40 bis 60 Gew. % bezogen auf die Gesamtmenge Salvia officinalis L. umfasst.

Insbesondere bevorzugt ist eine Zusammensetzung, die einen Anteil von Calendulae flos von 50 bis 55 Gew. % bezogen auf die Gesamtmenge Calendula officinalis L., von Matricariae flos von 50 bis 55 Gew. % bezogen auf die Gesamtmenge Matricaria recutita L. und von Salviae officinalis folium von 50 bis 55 Gew. % bezogen auf die Gesamtmenge Salvia officinalis L. umfasst.

Neben dem Anteil an Calendulae flos, Matricariae flos, Salviae officinalis folium in der erfindungsgemäßen Zusammensetzung spielt auch die pharmazeutische Qualität, d.h. Identität, Reinheit und Gehalt der verwendeten Pflanzen bzw. Arzneidrogen eine entscheidende Rolle.

Es ist bekannt, dass die Arzneidrogen-Qualität bezüglich der Zusammensetzung und dem Mengenverhältnis der einzelnen Inhaltsstoffe einerseits vom Standort, von der Länge der Vegetationsperiode, von den klimatischen Verhältnissen, aber auch vom Erntezeitpunkt, der Lagerung und zahlreichen weiteren Faktoren abhängig ist. Geringe Veränderungen können zu drastischen Variationen im Inhaltsstoffspektrum des Pflanzenmaterials führen.

Um Schwankungen der Arzneidrogen-Qualität festzustellen, ist es notwendig, die Arzneidrogen auf Qualität und Quantität ihrer Inhaltsstoffe zu überprüfen. Ist der Gehalt der einzelnen Inhaltsstoffe - insbesondere der potentiellen Wirkstoffe - bestimmt, kann eine Therapie mit Arzneidrogen, hinsichtlich einer Erhöhung oder einer Reduktion des eingesetzten Pflanzenmaterials beziehungsweise hinsichtlich der Häufigkeit der Anwendungen, an die Ergebnisse der Gehaltsbestimmung angepasst werden.

Neben der Bestimmung der Menge an Inhaltsstoffen ist für die Charakterisierung der Droge eine Prüfung auf Identität ebenso wichtig.

Daher wurden die verwendeten Pflanzen bzw. ihre Arzneidrogen vor ihrer weiteren Verarbeitung hinsichtlich ihrer Identität und ihres Wirkstoffgehaltes überprüft.

Die Prüfung auf Identität erfolgt sowohl visuell (visuelle Pflanzenbestimmung), als auch durch physikalische/ physiko-chemische Methoden, d.h., es werden für die jeweilige Pflanze charakteristische

Inhaltsstoffe nachgewiesen, dabei genügt der Nachweis einiger weniger Substanzen. Hierfür sind die aus der Literatur bekannten analytischen Methoden wie zum Beispiel High Pressure Liquide Chromatographie (HPLC), Gaschromatographie (GC) und Dünnschichtchromatographie (DC) geeignet.

Bei der visuellen Bestimmung für die jeweilige Pflanze bzw. Arzneidroge sind folgende Kriterien zu beachten:

Calendulae flos zeichnet sich dadurch aus, dass die Zungenblüten aus einer gelben oder orangegelben, ca. 3 bis 5 mm, im Mittelabschnitt etwa 7 mm breiten, an der Spitze dreizähnigen Zunge, einer behaarten, teilweise sichelförmigen, gelblichbraunen bis orangebraunen mit herausragendem Griffel und zweiteiliger Narbe sowie gelegentlich noch mit einem teilweise gekrümmten, gelblichbraunen bis orangebraunen Fruchtknoten bestehen. Die vorhandenen etwa 5 mm langen Röhrenblüten bestehen aus einer gelben, orangenroten oder rotvioletten, fünflappigen Blumenkrone und einer gelblichbraunen oder orangebraunen Röhre, die im unteren Teil behaart ist und der meist noch der teilweise gekrümmte, gelblichbraune bis orangebraune Fruchtknoten anhaftet.

Die Hüllkelchblätter von Matricariae flos sind verkehrt eiförmig bis lanzettich und haben einen häutigen, bräunlichgrauen Rand. Der Blütenboden, im wesentlichen kegelförmig, ist hohl und ohne Spreublättchen. Die Krone der Zungenblüten besteht aus einem basalen, hellgelben bis bräunlichgelben, röhrigen Teil, der in eine weiße, gestreckt eiförmige Zunge übergeht. Die Krone der Röhrenblüten ist gelb und erweitert sich nach oben, wo sie in 5 Zipfeln ausläuft; am Grund ist sie gelblich braun bis braun gefärbt.

Die Blattspreite des ganzen Salbeiblattes ist etwa 2 bis 10 cm lang und 1 bis 2 cm breit sowie länglich-eiförmig bis elliptisch. Der Blattrand ist fein gekerbt bis glatt. Die Blattspitze ist abgerundet oder kurz zugespitzt, das Blatt am Grunde in den Blattstiel verschmälert, abgerundet oder herzförmig. Die Blattoberseite ist grünlich grau und feinkörnig, die Blattunterseite weiß behaart und zeigt ein dichtes Netzwerk hervortretender Äderchen.

Alle drei Pflanzenarten zeichnen sich durch ein breites Spektrum an Inhaltsstoffen aus. Im Nachfolgenden werden die wichtigsten Gruppen genannt. Die dabei zitierten Konzentrationsangaben der Inhaltsstoffe entsprechen den in der Literatur geforderten Angaben (Hänsel R. et al., Hagers Handbuch der Pharmazeutischen Praxis, Springer Verlag, 5. Aufl., Band 4, 1992, S. 817-831, 597-615; Band 6, 1994, S. 538-574; Wichtl M., Teedrogen und Phytopharmaka: Ein Handbuch für die Praxis wissenschaftlicher Grundlage, 4. Aufl., 2002, Stuttgart: Wiss. Verl.-Ges., S.100-103. 369-373, 538-574; Hänsel R. et al., Pharmakognosie - Phytopharmazie, 7. Aufl., 1999, Springer Verlag, S. 524-526, 699-704, 740-743). Werden diese Werte bei den zu untersuchenden Pflanzendrogen erreicht, spricht man von Arzneibuchqualität.

Das Inhaltsstoffspektrum von Calendulae flos ist durch folgende Stoffgruppen gekennzeichnet:

- Triterpensaponine: Die als Saponoside beziehungsweise Calenduloside A-F bezeichneten Triterpensaponine stellen einfach gebaute Glykoside der Oleanolsäure dar. Ihr Gehalt wird in der Literatur mit 2-10 % angegeben (Hänsel R., Sticher O., Steinegger E., 1999, Pharmakognosie - Phytopharmazie, 7. Aufl., Springer Verlag, 524-526, 699-704, 740-743).

- Triterpenalkohole: Sie zählen zu den Hauptwirkstoffen der Calendula officinalis L.. Ein Gemisch von überwiegend acylierten pentacyclischen Mono-, Di- und Trihydroxytriterpenen kann in den Blüten nachgewiesen werden. Alle Alkohole kommen frei oder verestert vor. Während bei den Mono-Alkoholen nur etwa 10 % verestert sind, liegt der veresterte Anteil der Diole bei 98 %. Als Säure dient zur Veresterung bei den Mono-Alkoholen die Essigsäure, bei den Diolen hingegen Laurin-, Myristin-und Palmitinsäure. Die freien Mono-Alkohole sind aus pharmazeutischer Sicht weniger aktiv als die Diole. Es existieren 98 % der Diolester als 3-Monoester, jedoch nur 2 % als Diester. Die 3-Monoester liegen in den getrockneten Blüten zu ca. 4 % vor (Hänsel R., Keller K., Rimpler H., Hagers Handbuch der Pharmazeutischen Praxis, Springer Verlag, 5. Aufl., Band 4, 1992, 817-831, 597-615, Band 6, 1994, 538-574). Für die entzündungshemmende Wirkung werden vor allem freie und veresterte Mono-Alkohole vom Taraxasterol-Typ ebenso wie Diole vom Faradiol- und Arnidiol-Typ verantwortlich gemacht. Die aktivste Substanz bei den Mono-Alkoholen ist ψ-Taraxasterol, bei den Diolen Faradiol, wobei die Hauptbestandteile bei den Faradiolestern Faradiollaurat, Faradiolmyristat und Faradiolpalmitat sind.

- Sterole: Der Gehalt an Sterolen beträgt in den getrockneten Blüten 0,06-0,08 %. Sie kommen sowohl als freie Alkohole, aber ebenso als Ester und Glykoside vor.

- Flavonoide: Von 0,3 bis 0,8 % reicht der Gehalt an Flavonoiden (berechnet als Hyperosid ($C_{21}H_{20}O_{12}$; $M_r$ 464,4)), die vorwiegend aus Isorhamnetin- und Quercetinglykosiden zusammengesetzt sind.

- Carotinoide: Auf der Anwesenheit von Carotinoiden (bis zu 1,5 %) beruht die Farbe der Calendulablüten. Während sich orange blühende Varietäten durch einen hohen Anteil an Carotinen auszeichnen, überwiegen in gelb gefärbten Sorten die Xanthophylle. Als Hauptbestandteile sind in beiden Varietäten Lutein und Zeaxanthin zu nennen.

- Weitere Bestandteile: Ätherisches Öl (0,2 %), Polysaccharide (etwa 14 %) und Cumarine.

Unter einem ätherischen Öl versteht man eine Mischung aus fettlöslichen, leicht flüchtigen Substanzen, die überwiegend aus der Gruppe der Terpenoide stammen, und einem Duftstoff, der für die jeweilige Pflanze aus der das ätherische Öl gewonnen wurde, charakteristisch ist. Sie unterscheiden sich von herkömmlichen pflanzlichen Ölen (z.B. Sonnenblumenöl) dadurch, dass sie vollständig verdampfen (ätherisch = flüchtig, leicht verdampfend) und auf Papier keinen charakteristischen "Fettfleck" hinterlassen - man nennt sie deshalb auch "trocknende Öle". Jedem ätherischen Öl kann eine spezifische Pflanze zugeordnet werden.

Relevante Substanzen in Matricariae flos sind: (Hänsel R. et al., Hagers Handbuch der Pharmazeutischen Praxis, Springer Verlag, 5. Aufl., Band 4, 1992, S. 817-831, 597-615; Band 6, 1994, S. 538-574; Wichtl M., Teedrogen und Phytopharmaka: Ein Handbuch für die Praxis wissenschaftlicher Grundlage, 4. Aufl., 2002, Stuttgart: Wiss. Verl.-Ges., S.100-103. 369-373, 538-542; Hänsel R. et al., Pharmakognosie - Phytopharmazie, 7. Aufl., 1999, Springer Verlag, S. 524-526, 699-704, 740-743)

- Ätherisches Öl (Matricariae aetheroleum): Der Gehalt der Droge an ätherischem Öl beträgt 0,3 bis 1,5 %, wobei die Zusammensetzung - abhängig von Provenienz und Sorte - etwas wechseln kann. Zu den Bestandteilen des Kamillenöls zählen die Bisaboloide, bei denen das (-)-α-Bisabolol mengenmäßig dominiert. Durch Oxidationsvorgänge werden in der lebenden Pflanze die Bisabolol- und Bisabolonoxide gebildet, bei denen zwischen (-)-α-Bisaboloxid A, B und C sowie dem (-)-α-Bisabolonoxid A unterschieden werden muss. Bedeutend ist zudem der tiefblau gefärbte, nicht genuine Wirkstoff Chamazulen, der erst im Laufe der Wasserdampfdestillation aus Matricin, einem trizyklischen Sesquiterpenlakton vom Guajanolidtyp, entsteht. Außerdem konnten cis/trans-En-In-Dicycloether nachgewiesen werden.

- Flavonoide: Für die antiphlogistische Wirkung werden neben dem ätherischen Öl vor allem die Flavonoidmonoglycoside, acetylierte Flavonmonoglycoside und ihre Aglyca verantwortlich gemacht. Insgesamt macht ihr Gehalt bis zu 5 % aus. Man findet speziell Derivate des Apigenins, des Luteolins und Quercetins.

- Cumarine: Herniarin und Umbelliferon sind im Durchschnitt zu 0,06 beziehungsweise zu 0,01 % in Matricariae flos enthalten.

- Weitere Bestandteile: Polysaccharide (bis zu 10 %), Phenolcarbonsäuren.

Die wichtigsten Inhaltsstoffgruppen der Arzneidroge Salviae officinalis folium sind: (Hänsel R. et al., Hagers Handbuch der Pharmazeutischen Praxis, Springer Verlag, 5. Aufl., Band 4, 1992, S. 817-831, 597-615; Band 6, 1994, S. 538-574; Wichtl M., Teedrogen und Phytopharmaka: Ein Handbuch für die Praxis wissenschaftlicher Grundlage, 4. Aufl., 2002, Stuttgart: Wiss. Verl.-Ges., S.100-103. 369-373, 538-542; Hänsel R. et. al., Pharmakognosie - Phytopharmazie, 7. Aufl., 1999, Springer Verlag, S. 524-526, 699-704, 740-743)

- Ätherisches Öl: Der Gehalt beträgt in Salviae officinalis folium 1 bis 2,5 %. Die Zusammensetzung des ätherischen Öls variiert allerdings stark, weshalb auch die Bestimmung der Mengenverhältnisse einzelner Bestandteile zueinander zwecks einer Unterscheidung zwischen Salvia officinalis L. und Salvia triloba L. fil. (Griechischer Salbei) wichtig ist. Während bei Salvia officinalis L. die Summe der Anteile an $\alpha$-Thujon, $\beta$-Thujon und Campher zusammen etwa 70 % ergibt, sind diese drei Bestandteile im ätherischen Öl von Salvia triloba L. fil. zu maximal 13 % enthalten. Charakteristisch für den griechischen Salbei ist hingegen der hohe Gehalt an 1,8-Cineol (ca. 70 %).

- Gerbstoffe: Ihr Gehalt beträgt 3 bis 7 %, wovon 2 bis 3 % auf Rosmarinsäure entfallen.

- Bitterstoffe: In der Schnittdroge Salviae officinalis folium können 0,35% an Bitterstoffen nachgewiesen werden. Als Träger des bitteren Geschmacks gilt Carnosol (Pikrosalvin).

- Triterpene: Mit einem Gehalt von 2,5 bis 8 % ist Ursolsäure unter den Triterpenen quantitativ am bedeutendsten.

- Weitere Bestandteile: Flavonoide (1 bis 3 %, Luteolin u. a.) sowie Spurenelemente.

Ist die visuelle Bestimmung auf Identität positiv, so lässt sich durch den Nachweis der wichtigsten Inhaltsstoffe die Identität der Pflanzen eindeutig belegen und damit die gewünschte Zusammensetzung charakterisieren.

Demnach ist eine Ausführungsform der Zusammensetzung bevorzugt, bei der Calendulae flos Chlorogensäure, Rutin, Hyperosid und Isorhamnetin-3-O-rutinosid umfasst.

Des weiteren ist eine Ausführungsform bevorzugt, bei der Matricaria flos Herniarin, En-In-Dicycloether und $\alpha$-Bisabolol umfasst.

Außerdem ist eine Ausführungsform bevorzugt, bei der Salviae officinalis folium Cineol, Thujon und Bornylactat umfasst.

Eine weitere bevorzugte Ausführungsform ist die, bei der das ätherische Öl von Salviae officinalis folium Cineol, Thujon und Campher umfasst.

Dabei ist besonders eine Ausführungsform bevorzugt, bei der die Menge an Thujon und Campher jeweils den Cineolanteil im ätherischen Öl von Salviae officinalis folium übertrifft und insbesondere, wenn der Gehalt an Thujon und Campher mindestens 65 Gew.% beträgt, bezogen auf die Menge an ätherischem Öl in Salvia officinalis folium.

Um die gewünschte pharmazeutische Qualität zu gewährleisten, ist, wie bereits erwähnt, neben der Identität auch der Gehalt bestimmter Inhaltsstoffe ausschlaggebend. Dabei wäre es natürlich wünschenswert, wenn die Arzneidrogen Arzneibuchqualität aufwiesen. Es hat sich jedoch gezeigt, dass die erfindungsgemäße Zusammensetzung auch bei einer geringeren Konzentration an bestimmen Inhaltsstoffen die gewünschte pharmazeutische Wirkung aufweißt.

Demnach ist eine Ausführungsform bevorzugt, bei der der Faradiolesteranteil in der Gesamtmenge Calendulae flos mindestens 0,6 Gew.% umfasst, bevorzugter ist ein Anteil von mindestens 0,64 Gew.%, mehr bevorzugt von mindestens 0,85 Gew.%, noch mehr von mindestens 0,98 Gew.%, besonders bevorzugt von mindestens 1,2 Gew.%, insbesondere bevorzugt von mindestens 2,9 Gew.% und am meisten bevorzugt von 4,2 Gew.%.

Ein bevorzugter Bereich für den Gehalt an Faradiolestern in der Gesamtmenge Calendulae flos ist 0,6 bis 4,2 Gew.%, mehr bevorzugt ist der Bereich von 0,8 bis 3,0 Gew.% und noch mehr bevorzugt von 0,6 bis 1,2 Gew.%.

Bevorzugt ist außerdem eine Ausführungsform, bei der die Faradiolester mindestens Faradiollaurat, Faradiolmyristat und Faradiolpalmitat umfassen.

Bevorzugt ist des weiteren eine Ausführungsform, bei der in der Gesamtmenge Matricariae flos mindestens 0,2 Gew.% ätherisches Öl enthalten ist, mehr bevorzugt ist ein Anteil an ätherischem Öl in der Gesamtmenge von Matricariae flos von mindestens 0,21 Gew.%, noch mehr bevorzugt von mindestens 0,23 Gew.%, besonders bevorzugt von mindestens 0,31 Gew. %, insbesondere bevorzugt von mindestens 0,34 Gew.%, und am meisten bevorzugt von 1,5 Gew.%.

Ein bevorzugter Bereich an Gehalt an ätherischem Öl in der Gesamtmenge Matricariae flos ist 0,2 bis 1,5 Gew. %, mehr bevorzugt ist ein Bereich von 0,2 bis 0,5 Gew.% und besonders bevorzugt von 0,2 bis 0,35 Gew.%.

Außerdem bevorzugt ist eine Ausführungsform, bei der der Gehalt an ätherischem Öl in der Gesamtmenge Salviae officinalis folium mindestens 0,6 Gew.% umfasst, mehr bevorzugt ist ein Gehalt von mindestens 0,68 Gew.%, noch mehr von mindestens 0,80 Gew.%, besonders bevorzugt von mindestens 0,83 Gew.%, insbesondere bevorzugt von mindestens 0,89 Gew.% und am meisten bevorzugt von 2,5 Gew.%.

Ein bevorzugter Gehaltsbereich an ätherischem Öl in der Gesamtmenge Salviae officinalis folium ist 0,6 bis 2,5 Gew.%, mehr bevorzugt ist ein Bereich von 0,6 bis 0,9 Gew.% und noch mehr bevorzugt von 0,6 bis 0,7 Gew.%.

Bevorzugt ist außerdem eine Ausführungsform, bei der der Rosmarinsäuregehalt in der Gesamtmenge Salviae officinalis folium mindesten 0,6 Gew.% umfasst, mehr bevorzugt ist ein Gehalt von mindestens 0,62 Gew.%, noch mehr bevorzugt von mindestens 0,77 Gew.% und am meisten bevorzugt von 1,7 Gew.%.

Ein bevorzugter Bereich an Rosmarinsäuregehalt in der Gesamtmenge Salviae officinalis folium ist 0,6 bis 1,7 Gew.%, mehr bevorzugt ist ein Bereich von 0,6 bis 0,9 Gew.% und noch mehr bevorzugt von 0,6 bis 0,8 Gew.%.

Um eine einfache therapeutische Anwendung der erfindungsgemäßen Zusammensetzung für den Patienten zu gewährleisten, werden entweder Extrakte (z.B. Dekokte, Infuse, Extracta fluida, Extracta spissa, Extracta sicca, Extracta tenua, Extractum aquosum siccatum, Extractum spirituosum siccatum), Tinkturen, Salben, Cremes, Emulsionen oder Gele der Zusammensetzung hergestellt.

Extrakte sind konzentrierte Zubereitungen, die durch Auslaugen der zerkleinerten Drogenmaterialien gewonnen werden. Dabei werden die Drogenwirkstoffe unter Einwirkung einer Flüssigkeit (Auszugsmittel oder Extraktionsmittel) in die Lösung überführt. Die Herstellung der Extrakte kann durch Mazeration oder Perkolation erfolgen.

Die Mazeration ist das einfachste Auszugsverfahren für Drogen und wird auch als Gleichgewichtsextraktion bezeichnet. Das zerkleinerte Drogenmaterial wird dabei mit dem Extraktionsmittel versetzt und so längere Zeit (bis zu mehreren Tagen) unter Umschütteln aufbewahrt. Dabei kommt es zu einer Gleichgewichtseinstellung zwischen Extraktionsmittel und den extrahierbaren Inhaltsstoffen. Beim Einsatz konstanter Drogenmenge nimmt demnach die Menge an extrahierten Pflanzeninhaltsstoffen mit zunehmender Extraktionsmittelmenge zu.

Bei der Perkolation, die auch als erschöpfende Extraktion bezeichnet wird, wird im Unterschied zur Mazeration so lange extrahiert bis alle Inhaltsstoffe in den Extrakt übergegangen sind. Um dies zu erreichen, wird das zerkleinerte Drogenmaterial gründlich mit einem Anteil der vorgeschriebenen Extraktionsflüssigkeit gemischt, eine angemessene Zeit lang stehen gelassen und anschließend in einen Perkolator gefüllt. Das Perkolat wird bei Raumtemperatur so langsam abtropfen gelassen, dass die verbleibende Extraktionsflüssigkeit die zu extrahierende pflanzliche Droge stets bedeckt. Der Rückstand kann ausgepresst und die Pressflüssigkeit mit dem Perkolat vereinigt werden.

Wäßrige Drogenauszüge sind entweder Dekokte (Abkochungen) oder Infuse (Aufgüsse). Bei der Abkochung wird das Drogenmaterial mit kaltem Wasser versetzt und anschließend zum Sieden gebracht. Die festen Bestandteile werden anschließend abgeseiht. Beim Aufguss werden die getrockneten und zerkleinerten Drogenmaterialien mit kochendem Wasser übergossen und in einem geschlossenen Gefäß ziehen gelassen.

Extrakte sind Zubereitungen von flüssiger (Fluidextrakte, Extracta fluida und Tinkturen), zähflüssiger (Dickextrakte, Extracta spissa) oder fester (Trockenextrakte, Extracta sicca) Beschaffenheit, die aus üblicherweise getrockneten, pflanzlichen Drogen durch Perkolation und/oder Mazeration hergestellt werden (Ph. Eur. 4 Ausgabe, Grundwerk 2002, S 710-711 und Ph. Eu. 4. Ausgabe , 3. Nachtrag, S. 3765). Verschiedene Extrakttypen werden unterschieden. Standardisierte Extrakte werden innerhalb zulässiger Grenzen auf einen vorgegebenen Gehalt an bekannten wirksamkeitsbestimmenden Inhaltsstoffen eingestellt; die Einstellung erfolgt mit inerten Materialien oder durch Mischen von Extrakt-Chargen. Andere Extrakte werden im Wesentlichen durch ihr Herstellungsverfahren (Beschaffenheit der zu extrahierenden pflanzlichen Droge; Lösungsmittel; Extraktionsbedingungen) sowie ihre Spezifikationen definiert. Extrakte werden durch geeignete Methoden unter Verwendung von geeigneten Lösungsmitteln, d.h., Lösungsmittel, die in der Lage sind, die gewünschten Inhaltsstoffe aus dem Pflanzenmaterial zu lösen, wie Wasser oder organische Lösungsmittel, z.B. 70%iger wässriger Ethanol, Aceton, isoPropanol etc., hergestellt. Verschiedene Chargen der pflanzlichen Droge können vor der Extraktion gemischt werden. Die zu extrahierende pflanzliche Droge kann einer Vorbehandlung unterzogen werden, beispielweise der Inaktivierung von Enzymen, dem Zerkleinern oder dem Entfetten. Zusätzlich können unerwünschte Stoffe nach der Extraktion entfernt werden.

Fluidextrakte (Extracta fluida) sind flüssige Zubereitungen, von denen im Allgemeinen ein Masse- oder Volumenteil einem Masseteil der getrockneten pflanzlichen Droge entspricht (Ph. Eur. 4 Ausgabe, Grundwerk **2002**, S. 711 und Ph. Eu. 4. Ausgabe , 3. Nachtrag, S. 3766). Diese Zubereitungen werden falls erforderlich so eingestellt, dass sie den Anforderungen bezüglich Lösungsmittelgehalt und, falls zutreffend, Gehalt an Inhaltsstoffen entsprechen. Fluidextrakte werden durch Extraktion der pflanzlichen Droge unter Verwendung von organischen Lösungsmitteln, wie z.B. Ethanol, geeigneter Konzentration oder von Wasser hergestellt oder aber durch Lösen eines zähflüssigen Extraktes oder Trockenextrakts (ihrerseits hergestellt durch Verwendung der gleichen Extraktionsflüssigkeit mit gleicher Konzentration, wie sie bei der Herstellung des Fluidextraktes durch direkte Extraktion verwendet wird) der pflanzlichen Droge in einem Lösungsmittel geeigneter Konzentration. Falls erforderlich werden Fluidextrakte filtriert. Fluidextrakte können geeignete Konservierungsmittel enthalten.

Dünnflüssige Extrakte (Extracta tenua) sind Extrakte mit einer Restfeuchtigkeit von ca. 30 Prozent bezogen auf die Gesamtmenge Extrakt.

Zähflüssige Extrakte (Extracta spissa) sind halbfeste Zubereitungen, die durch Eindampfen oder teilweises Eindampfen des bei der Extraktion verwendeten Lösungsmittels hergestellt werden (Ph. Eur. 4 Ausgabe, Grundwerk 2002, S 711-712 und Ph. Eu. 4. Ausgabe , 3. Nachtrag, S. 3767). Sie besitzen eine Restfeuchtigkeit von ca. 20 Prozent bezogen auf die Gesamtmenge Extrakt.

Trockenextrakte (Extracta sicca) sind feste Zubereitungen, die durch Verdampfen des bei ihrer Herstellung verwendeten Lösungsmittels erhalten werden (Ph. Eur. 4 Ausgabe, Grundwerk 2002, S 712 und Ph. Eu. 4. Ausgabe , 3. Nachtrag, S. 3767). Trockenextrakte haben im Allgemeinen einen Trocknungsverlust oder einen Wassergehalt von höchstens 5 % (m/m), d.h. einen Trockenrückstand vom mindestens 95 % (m/m). Geeignete inerte Materialien können zugesetzt sein. Eingestellte Trockenextrakte sind auf einen definierten Gehalt an Inhaltsstoffen eingestellt. Dazu dienen geeignete inerte Hilfsstoffe oder ein Trockenextrakt des für die Herstellung verwendeten pflanzlichen Materials. Gegebenfalls schreibt die Monographie eines Trockenextraktes eine Grenzprüfung für das zur Herstellung verwendete Lösungsmittel vor. Die Reinheit eines Trockenextraktes kann wie z.B. im Österreichischen Arzneimittelbuch (ÖAB) (Pharmacopoea Austriaca, Band II, Spezieller Teil A - J, Amtliche Ausgabe 1990, 12. Nachtrag, Verlag Österreich) angegeben, bestimmt werden. Trockenextrakte sollten vor Licht geschützt in einem kleinen, dicht schließenden Gefäß mit geeignetem Trockenmittel aufbewahrt werden. Es ist zulässig, von Trockenextrakten auch Verreibungen mit Milchzucker im Verhältnis 1 : 1 vorrätig zu halten. Diese sind wie die Trockenextrakte aufzubewahren.

Tinkturen sind flüssige Zubereitungen, die aus getrocknetem pflanzlichem Material hergestellt werden (Ph. Eur. 4 Ausgabe, Grundwerk 2002, S 739-740 und Ph. Eu. 4. Ausgabe , 3. Nachtrag, S. 3766-3767). Bei einigen Zubereitungen muss das zu extrahierende Material einer Vorbehandlung unterzogen werden, beispielweise einer Inaktivierung von Enzymen, einem Zerkleinern oder einem Entfetten. Tinkturen werden durch Mazeration, Perkolation oder durch andere geeignete und validierte Methoden unter Verwendung von Ethanol geeigneter Konzentration hergestellt. Tinkturen können auch durch Lösen oder Verdünnen von Extrakten unter Verwendung von Ethanol hergestellt werden.

Wie bereits erwähnt, werden für die Behandlung von erythematösen Hautveränderungen aus der oben beschriebenen Zusammensetzung Extrakte oder Tinkturen hergestellt und bei den Patienten angewendet.

Mögliche Herstellungsarten, aber keineswegs ausschließliche, von solchen Extrakten und Tinkturen aus der erfindungsgemäßen Zusammensetzung sind z.B.:

Die Herstellung eines Aufgusses (Infus). Dabei wird die erfindungsgemäße Zusammensetzung zerkleinert sowie anschließend mit heißem Wasser übergossen, die Pflanzenrückstände abgetrennt und der so erhaltene wässrige Extrakt mit Wasser weiter verdünnt.

Zur Extraktion wird das Gemisch zugedeckt und unter teilweisem Umrühren ziehen gelassen, wobei die Ziehphase mindestens 10 Minuten betragen sollte.

Dabei ist jedes Mischungsverhältnis zwischen erfindungsgemäßer Zusammensetzung und Extraktionsmittel, z.B. Wasser, denkbar. Bevorzugt ist jedoch eine Ausführungsform des Verfahrens, bei der das Verhältnis der erfindungsgemäßen Zusammensetzung zu Wasser im so erhaltenen wässrigen Extrakt 1 : 45 umfasst, mehr bevorzugt ist ein Verhältnis von erfindungsgemäßer Zusammensetzung zu Wasser von 1 : 50, noch mehr von 1 : 80, insbesondere von 1 : 90 und am meisten bevorzugt von 1 : 100.

Besonders bevorzugt ist eine Ausführungsform, bei der das Verhältnis der erfindungsgemäßen Zusammensetzung zu Wasser in einem Bereich von 1 : 45 bis 1 : 100 liegt, und insbesondere, wenn der Extrakt 2 bis 4 Liter Wasser, 10 g Calendulae flos, 10 g Matricariae flos und 10 g Salviae officinalis folium umfasst.

Des weiteren ist eine Ausführungsform bevorzugt, wenn der so erhaltene wässrige Extrakt mit Wasser in einem Verhältnis von 35 : 65 Anteilen (Extrakt : Wasser) weiter verdünnt wird, noch mehr bevorzugt ist ein Verhältnis von 40 : 60, insbesondere bevorzugt von 45 : 55-und am meisten bevorzugt von 60 : 40.

Besonders bevorzugt ist eine Ausführungsform, bei der der wässrige Extrakt in einem Bereich von 40 bis 60 Anteilen mit 60 bis 40 Anteilen Wasser weiter verdünnt wird.

Die daraus resultierende Mischung kann dann zum Beispiel in Form von 15 bis 20 minütigen Hand- und Fußbädern am Patienten angewendet werden.

Trockenextrakte (Extracta sicca) können folgendermaßen zubereitet werden (ÖAB (Pharmacopoea Austriaca), Band II, Spezieller Teil A- J, Amtliche Ausgabe 1990, 12. Nachtrag, Verlag Österreich):

Die erfindungsgemäße Zusammensetzung ist im grob gepulvertem Zustand zu verarbeiten. Die Extraktion der Drogen erfolgt in der Regel nach dem Perkolationsverfahren, wobei jedoch das 1. Teilperkolat als Vorlauf gilt. Die gewonnen Perkolate und die filtrierte Pressflüssigkeit dampft man beginnend mit der Pressflüssigkeit und dem zuletzt erhaltenen Teilperkolat bei möglichst niedriger Temperatur unter vermindertem Druck zu Trockene ein.

Ist der Trockenextrakt auf einen bestimmten Gehalt an wirksamen Stoffen herzustellen, so kann dies nach zwei Methoden erfolgen:

1. Man dampft die Perkolate zunächst nur bis zur Konsistenz eines dünnen Extraktes ein. Nach der Feststellung des Gewichtes dieser Extraktbrühe bestimmt man den Gehalt an Wirkstoffen und den Trockenrückstand (ÖAB.: IX, 2. b. ζ) und versetzt nötigenfalls mit der erforderlichen Menge Milchzucker, Dextrin oder Rohrzucker (X in g). Diese berechnet man nach der folgenden Formel:

$$X = \frac{95 \cdot a}{b} - T$$

a = gefundener Gehalt an Wirkstoffen in der Extraktbrühe in g
b = geforderter Gehalt an Wirkstoffen im fertigen Trockenextrakt in %
T = Gewicht des Trockenrückstandes der Extraktbrühe in g

Nachdem man das Verdünnungsmittel in der Extraktbrühe gelöst hat, dampft man zur Trockene ein.
2. Man dampft die Perkolate bis zur Trockene ein und verreibt das Extrakt dann mit der erforderlichen Menge des Verdünnungsmittels, die man nach obiger Formel berechnet hat.

Das gewonnene Extrakt wird fein zerrieben und im Vakuumexsikkator nachgetrocknet. Die Aufbewahrung erfolgt wie oben beschrieben.

Eine Ausführungsform der Tinktur ist, wenn diese 1 Teil der erfindungsgemäßen Zusammensetzung und 10 Anteile 30 bis 70 Vol.%igen wässrigen Ethanol enthält, bevorzugter ist es, wenn die Tinktur die Zusammensetzung zu 1 Anteil und zu 9 Anteilen 30 bis 70 Vol.%igen wässrigen Ethanol in der Tinktur enthält, mehr bevorzugt ist es, wenn diese die Zusammensetzung zu 1 Anteil und zu 7 Anteilen 30 bis 70 Vol.%igen wässrigen Ethanol enthält, noch mehr bevorzugt, wenn diese die Zusammensetzung zu 1 Anteil und zu 6 Anteilen 30 bis 70 Vol.%igen wässrigen Ethanol enthält und am meisten bevorzugt ist, wenn die Tinktur die Zusammensetzung zu 1 Anteil und zu 5 Anteilen 30 bis 70 Vol.%igen wässrigen Ethanol in der Tinktur enthält.

Besonders bevorzugt ist eine Ausführungsform der Tinktur, wenn diese die erfindungsgemäße Zusammensetzung zu 1 Anteil und 30 bis 70 Vol.%igen wässrigen Ethanol zu 5 bis 10 Anteilen enthält.

Eine andere Therapiemöglichkeit ist die Verwendung von aus der erfindungsgemäßen Zusammensetzung hergestellten Salben, Cremes, Emulsionen oder Gele. Dabei werden zumeist die aus der erfindungsgemäßen Zusammensetzung hergestellten Extrakte und/oder Tinkturen in eine hydrophile oder ambiphile Salbengrundlage eingearbeitet.

Salben sind einphasige (z.B. Fettphase) halbfeste Arzneiformen, in der feste oder flüssige Substanzen dispergiert sind (Ph. Eur. 4 Ausgabe, Grundwerk 2002, S 752-753 und Ph. Eu. 4. Ausgabe, 3. Nachtrag, S. 3776-3777). Sie enthalten eine Salbengrundlage (Kohlenwasserstoff-, Hydrodispersions-, Lipo-, Hydro- oder Silicongel), die fein verteilte Wirkstoffe zur Anwendung an Haut- oder Schleimhäuten enthalten kann. Man unterscheidet je nach Wassergehalt hydrophobe, wasseraufnehmende und hydrophile Salben.

Hydrophobe Salben können nur kleine Mengen an Wasser aufnehmen. Typische Grundlagen für die Herstellung dieser Salben sind Hartparaffin, dickflüssiges und dünnflüssiges Paraffin, pflanzliche Öle, tierische Fette, synthetische Glyceride, Wachse und flüssige Polyalkylsiloxane.

Wasser aufnehmende Salben können größere Mengen Wasser aufnehmen und damit in Abhängigkeit von der Art des Emulgators Emulsionen vom Wasser-in-Öl-Typ oder vom Ö1-in-Wasser-Typ bilden. Zu diesem Zweck können Emulgatoren vom Wasser-in-Öl-Typ, wie Wollwachsalkohole, Sorbitanester, Monoglyceride und Fettalkohole, oder Öl-in-Wasser-Typ, wie sulfatierte Fettalkohole, Polysorbate, Macrogolcetostearylether oder Ester von Fettsäuren mit Macrogolen, verwendet werden. Grundlage der Wasser aufnehmenden Salben sind diejenigen der hydrophoben Salben.

Hydrophile Salben sind Zubereitungen, deren Grundlagen mit Wasser mischbar sind. Diese Salbengrundlagen bestehen üblicherweise aus Mischungen von flüssigen und festen Macrogolen (Polyethylenglycolen). Sie können Wasser in geeigneten Mengen enthalten.

Eine Creme ist eine mehrphasige, halbfeste Zubereitung aus einer lipophilen und einer wässrigen Phase (Ph. Eur. 4 Ausgabe, Grundwerk 2002, S 753 und Ph. Eu. 4. Ausgabe, 3. Nachtrag, S. 3777).

In lipophilen Cremes ist die äußere Phase lipophil. Sie enthalten Emulgatoren vom Wasser-in-Öl-Typ, wie zum Beispiel Wollwachsalkohole, Sorbitanester und Monoglyceride.

In hydrophilen Cremes ist die äußere Phase die wässrige Phase. Die Zubereitungen enthalten Emulgatoren

vom Öl-in-Wasser-Typ, wie Natrium- oder Trolaminseifen, sulfatierte Fettalkohole, Polysorbate oder Ester von Polyethoxyfettsäuren und Polyethoxyfettsäurealkoholen, falls erforderlich in Mischung mit Emulgatoren vom Wasser-in-Öl-Typ.

Emulsionen bestehen aus einer wässrigen polaren und einer öligen unpolaren Phase. Man unterscheidet wie bei den Cremes zwischen Wasser-in-Öl und Öl-in-Wasser-Emulsionen. Um die notwendige Stabilisierung bei der Herstellung von Emulsionen zu erreichen, werden ebenfalls Emulgatoren eingesetzt, die die Grenzflächenspannung zwischen den beiden Phasen herabsetzen.

Gele bestehen aus gelierten Flüssigkeiten, die mit Hilfe von Gelbildnern (Quellmittel) hergestellt werden. Man unterscheidet zwischen hydrophoben Gelen und hydrophilen Gelen.

Hydrophobe Gele (OLEOGELE) sind Zubereitungen, deren Grundlage üblicherweise aus Paraffin mit Zusatz von Polyethylen oder aus fetten Ölen, die durch Zusatz von Siliciumdioxid, Aluminium- oder Zinkseifen geliert werden, besteht.

Hydrophile Gele (HYDROGELE) sind Zubereitungen, die üblicherweise aus Wasser, Glycerol oder Propylenglykol bestehen, die mit geeigneten Gelbildnern wie Stärke, Cellulosederivate, Carbomeren oder Magnesium-Aluminium-Silikaten, geliert werden.

Mögliche Ausführungsformen sind die Herstellung und Verwendung der Zusammensetzung in hydrophilen und ambiphilen Cremes oder Öl-in-Wasser-Emulsionen. Dabei werden zunächst Extrakte oder Tinkturen der erfindungsgemäßen Zusammensetzung hergestellt, die dann in hydrophile oder ambiphile Cremes, Salben, Gele oder Emulsionen eingearbeitet werden

Die so hergestellte Zusammensetzung bzw. deren Extrakt, Tinktur, Salbe, Creme, Emulsion oder Gel kann zur Herstellung eines Arzneimittels verwendet werden.

Besonders bevorzugt können sie zur Herstellung eines Arzneimittels zur Behandlung von erythematösen Hautveränderung verwendet werden.

Insbesondere können sie zur Herstellung eines Arzneimittels zur Behandlung von akralen erythematösen Hautveränderungen verwendet werden.

Um die Effektivität der Behandlung mit der erfindungsgemäßen Zusammensetzung zu überprüfen, wurde eine Studie mit 40 Patienten durchgeführt, die alle mit dem Zytostatikum Capecitabin (Handelsname Xeloda®) behandelt wurden und einen unterschiedlichen Schweregrad der erythematösen Hautveränderung aufwiesen. Bei den meisten Patienten kam es zu einer Verbesserung oder Heilung des Hand-Fuß-Syndroms nach der Behandlung mit der Zusammensetzung. Darüber hinaus konnte gezeigt werden, dass die Zusammensetzung nebenwirkungsfrei, einfach anzuwenden ist und eine hohe Akzeptanz bei den Patienten besitzt.

Die Erfindung wird an Hand der nachstehenden Abbildungen näher erläutert; es zeigen:

| | |
|---|---|
| **Abbildung 1:** | Das Gaschromatogramm der Inhaltstoffe des bei der Wasserdampfdestillation erhaltenen ätherischen Öls von Maricaria recutita L. |
| **Abbildung 2:** | Das Gaschromatogramm der Inhaltstoffe des bei der Wasserdampfdestillation erhaltenen ätherischen Öls von Salvia officinalis L. |
| | Peak: A: α-Pinen |
| | B: Camphen |
| | C: β-Pinen |
| | D: Cineol |
| | E: Thujon |
| | F: Campher |
| | G: Bornylacetat |
| **Abbildung 3:** | Das HPL-Chromatogramm von "Calendula-Blüte" Ernte 2001 |
| | Peaks: 1a: Lupeolacetat |
| | 2a: Faradiollaurat |
| | 3a: Faradiolmyristat |
| | 4a: Faradiolpalmitat |
| **Abbildung 4:** | Das HPL-Chromatogramm von "Calendula-Blüte" Ernte 2002 |
| | Peaks: 1b: Lupeolacetat |
| | 2b: Faradiollaurat |
| | 3b: Faradiolmyristat |
| | 4b: Faradiolpalmitat |
| **Abbildung 5:** | Das HPL-Chromatogramm eines aufbereiteten Extraktes von Salvia officinalis L. (Ernte 2002); |
| | Peak: 1: Rosmarinsäure |
| **Abbildung 6:** | Einteilung der erythematösen Hautveränderung in Schwergrade vor und nach der Behandlung mit der erfindungsgemäßen Zusammensetzung |

□ = Vorher

■ = Nachher

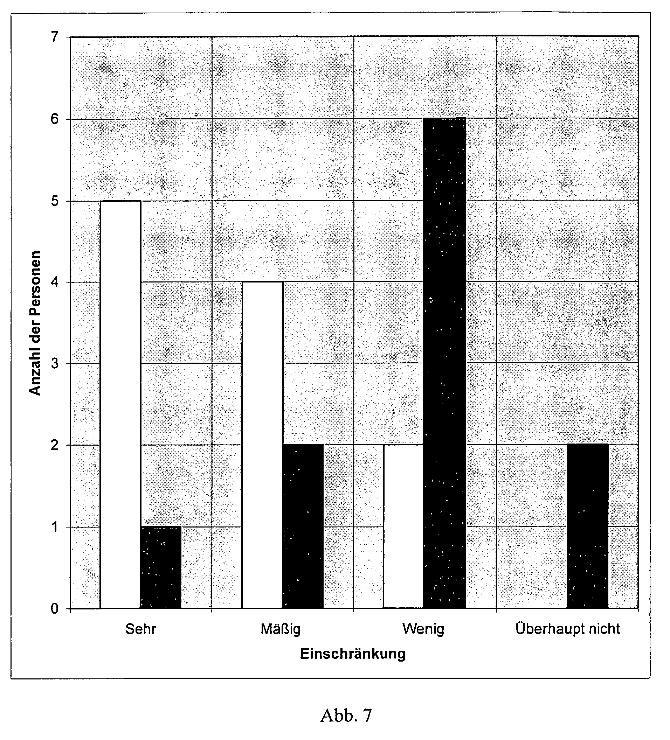

**Abbildung 7:** Einschränkung der täglichen Beschäftigung durch die erythematöse Hautveränderung vor und nach der Behandlung mit der erfindungsgemäßen Zusammensetzung

□ = Vorher

■ = Nachher

Die verwendeten Pflanzenmaterialien wurden alle von der Firma Waldland®, Vermarktungs Ges.m.b.H. Waldhof, und zwar die Ernten 2001 und/oder 2002 bezogen.

## A. Bestimmung der Identität

**Beispiel 1A:** Calendula officinalis L.

Bei Calendula officinalis L. wurden jeweils zwei Proben aus unterschiedlichen Erntejahren (2001 und 2002) untersucht, zum einen eine im Folgenden "Calendula-Mischung" genannte Probe, die neben Zungen- und Röhrenblüten auch Teile des Körbchenbodens, Samen und Stängelanteile enthielt, und eine Probe, im Folgenden "Calendula-Blüten" genannt, die reine Zungenblüten (Calendulae flos) enthielt.

Um den Anteil der Zungenblüten beziehungsweise das Ausmaß an Verunreinigungen zu bestimmen, wurden die Proben nach den einzelnen Bestandteilen geordnet. Es konnte ein Verhältnis von Zungenblüten zu anderen Pflanzenbestandteilen von etwa 50 : 50 ermittelt werden. Bei der Ernte 2002 wurde ein Anteil an Calendulae flos unter 50% festgestellt, daher wurde die Drogenmenge verdoppelt, um der Qualitätsminderung entgegenzuwirken.

Aufbereitung der Droge und Identitätsprüfung mittels DC wurden nach Wagner H.; Bladt S., 1996, A Thin Layer Chromatography Atlas, 2. Aufl., Springer-Verlag, 216 - 217, durchgeführt.

Parameter für die Dünnschichtchromatographie:

Stat. Phase: KG 60 $F_{254}$ Fertigplatte Merck, 20 x 20 cm

Mob. Phase: Ethylacetat - Ameisensäure - konz. Essigsäure - Wasser (100+11+11+26)

Untersuchungslösung:

1,0 g Drogenmaterial wurde mit 10 ml Methanol in einem Wasserbad für 15 Minuten unter Rückfluss erhitzt. Nach Abkühlung und anschließender Filtration wurden 20 μl der Extraktlösung auf die Dünnschichtchromatographieplatte aufgetragen.

Vergleichssubstanzen:

1 mg Rutin, Chlorogensäure, Hyperosid, Isorhamnetin-3-O-rutinosid wurden jeweils in 100 μl Methanol gelöst und 5 bis 10 μl davon auf die Dünnschichtchromatographieplatte aufgetragen.

Detektion:

Die entwickelte und getrocknete Dünnschichtplatte wurde mit Naturstoff-Reagens A (1%ige Lösung von Diphenylborsäure-β-aminoethylester in Methanol) besprüht. Nach erneutem Trocknen besprüht man die Platte mit Polyethylenglycol (PEG 400, 5 % in Ethanol) und betrachtet das Chromatogramm unter $UV_{3650}$.

Ergebnisse:

Es traten bei den Chromatogrammen der Untersuchungslösungen "Calendula-Mischung" und "Calendula-Blüte" unter $UV_{365}$ einige intensiv gefärbte Zonen auf, die sich hinsichtlich der Retentionszeitwerte ($R_f$-Werte) mit jenen der Referenzsubstanzen vergleichen ließen. Gut zu erkennen war im mittleren Teil der Chromatogramme die hellblau fluoreszierende Zone der Chlorogensäure. Die Zonen von Rutin, Hyperosid und Isorhamnetin-3-O-rutinosid ließen sich ebenso deutlich abgrenzen. Bei den Chromatogrammen von "Calendula-Mischung" und "Calendula-Blüte" bestand ein Unterschied bezüglich der Fluoreszenzintensität der einzelnen Zonen. Die stärkere Färbung im Chromatogramm von "Calendula-Blüte" ergab sich aus dem relativ höheren Prozentsatz an Zungenblüten im Vergleich zur Probe "Calendula-Mischung".

Die Proben "Calendula-Mischung" und "Calendula-Blüte" (Ernte 2001 und 2002) entsprachen der Prüfung auf Identität.

**Beispiel 2A:** Matricaria recutita L

Wie vom Europäischen Arzneibuch (EuAB, 1997, 129 - 130 und 1161 - 1162) gefordert, wiesen die zu untersuchenden Drogenproben der Firma Waldland® aus den Erntejahren 2001 und 2002 von Matricariae flos einen aromatischen, angenehmen und charakteristischen Geruch auf, wobei die einzelnen Proben sich hinsichtlich der relativen Zusammensetzung der einzelnen Bestandteile unterschieden. In manchen Proben konnte ein dominierender Anteil an Kamillengrus erfasst werden.

Bei der Drogenaufbereitung und Identitätsprüfung mittels DC wurde nach dem Europäischen Arzneibuch (EuAB. 1997, 129 - 130 und 1161 - 1162) vorgegangen.

Parameter für die Dünnschichtchromatographie:

Stat. Phase: KG 60 $F_{254}$ Fertigplatte Merck, 5 x 10 cm

Mob. Phase: Chloroform - Toluol (75+25)

Untersuchungslösung:

1. Wasserdampfdestillation:

2,0 g zerkleinerte Droge wurden mit 10 ml Wasser bis zum Siedepunkt erhitzt. Es wurden langsam ca. 0,9-1,1 ml überdestilliert. Das abgekühlte Destillat wurde mit 2 ml Petrolether ausgeschüttelt und anschließend die organische Phase mittels eines Rotationsverdampfers entfernt. Der Rückstand wurde in 0,1 ml Toluol aufgenommen und 20 $\mu$l auf die Dünnschichtplatte aufgetragen.

2. Perkolation: 1,0 g Droge wurde grob zerstoßen und danach in ein Glasrohr von 15 cm Länge und 1,5 cm Durchmesser mit grobporiger Fritte eingefüllt und mit Hilfe eines Glasstabes eingestampft. Anschließend wurden Mörser und Pistill zweimal mit je 10 ml Dichlormethan gewaschen und die Waschflüssigkeit nach und nach auf die Säule gegeben. Ungefähr 15 ml Perkolat wurden aufgefangen und das Lösungsmittel unter Vakuum entfernt. Der Rückstand wurde in 1,0 ml Toluol aufgenommen und 5 $\mu$l der zu untersuchenden Probe davon auf die Dünnschichtplatte aufgetragen.

Vergleichslösung:

0,10 g Borneol, 80 mg Bornylacetat und 4 mg Guajazulen wurden in Toluol zu 10,00 ml gelöst und 5 bzw. 10 $\mu$l aufgetragen.

Detektion: Betrachten unter $UV_{254}$ und $UV_{365}$.

Danach wurde die Platte mit einem frisch bereiteten Anisaldehyd-Schwefelsäure-Reagens (0,5 ml Anisaldehyd, 10 ml Eisessig, 5 ml konz. Schwefelsäure, 85 ml Methanol) besprüht und für 5 Minuten im Trockenschrank bei 100°C erhitzt.

Ergebnisse:

Unter $UV_{365}$ konnten auf dem Chromatogramm der Untersuchungslösung auf der Höhe von Borneol eine intensiv blau fluoreszierende Zone beobachtet werden (Herniarin). Man erkannte außerdem eine Reihe von fluoreszenzlöschenden Zonen unter $UV_{254}$. Die breiteste davon (En-In-Dicycloether) befand sich auf derselben Höhe wie die Zone des Bornylacetats im Chromatogramm der Referenzlösung. Nach dem Besprühen des Chromatogramms zeigten sich mehrere gefärbte Zonen.

Etwas über der Zone von Herniarin konnte man eine blauviolette Zone erkennen, die dem $\alpha$-Bisabolol zuzuordnen war. Olivgrün gefärbt, befand sich die En-In-Dicycloether-Zone auf derselben Höhe wie Bornylacetat. Gleichen $hR_F$-Wert wie Guajazulen zeigte eine blau gefärbte Zone des Chamazulens.

Der aus dem Drogenmaterial zur Untersuchung hergestellte Extrakt entsprach sowohl für die Ernte 2001 als auch 2002 der Prüfung auf Identität.

Zwecks weiterer Charakterisierung der Droge wurde eine gaschromatographische Analyse durchgeführt. Ziel war die Erstellung eines Fingerprints für Matricariae flos. Zusätzlich wollte man Auskunft über die Größenordnung der einzelnen Bestandteile erlangen.

Parameter für die GC-Analyse:

Gerät: Perkin-Elmer Auto System, FID

Säule: Kapillarsäule, 10 m x 0,25 mm i.D., Permabond OV-1-DF-0,50

Ofentemperatur: Programm 50 °C - 270 °C, 2,5 °C/Min. bei einer Initialzeit von 5 Minuten

Inj./Det. Temp.: 280 °C

Auswertung der GC-Analyse:

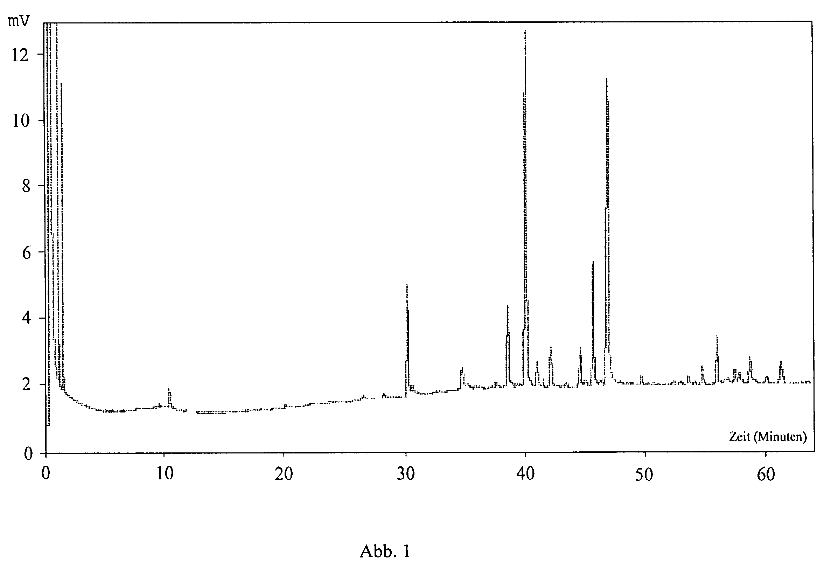

Das bei der GC erhaltene Chromatogramm (Abbildung 1) diente zur Erstellung eines Fingerprints. Es war nicht möglich, die Peaks bestimmten Substanzen näher zuzuordnen. Um die Identität der Kamillendroge dennoch über die Ergebnisse der GC belegen zu können, zog man ein Chromatogramm eines vorhandenen ätherischen Öls von Matricaria recutita L. als Vergleich heran (nicht gezeigt). Die Peaks beider Chromatogramme stimmten in den Retentionszeiten überein. Daher galt die Identität der Droge als bestätigt.

**Beispiel 3A:** Salvia officinalis L.

Die Proben von Salviae officinalis folium bestanden aus den geschnittenen, getrockneten Laubblättern von Salvia officinalis L..

Aufbereitung der Droge und Identitätsprüfung mittels DC wurden nach Wagner H.; Bladt S., 1996, A Thin Layer Chromatography Atlas, 2. Aufl., Springer-Verlag, 216 - 217 durchgeführt.

Parameter für die Dünnschichtchromatographie:

Stat. Phase: KG 60 $F_{254}$ Fertigplatte Merck, 5 x 10 cm

Mob. Phase: Toluol - Ethylacetat (93+7)

Untersuchungslösung:

1,0 g zerkleinerte Droge wurde mit 10 ml Wasser bis zum Siedepunkt erhitzt und langsam 2-3 ml überdestilliert. Nach dem Abkühlen des Destillats wurde mit 1 ml Dichlormethan oder Petrolether ausgeschüttelt und anschließend von der organischen Phase je 10 µl aufgetragen.

Vergleichslösung:

10 µl Cineol wurden in 5,0 ml Chloroform gelöst. Von dieser Lösung wurden 10 µl aufgetragen.

Als zweite Referenzlösung diente 5 µl eines auf Identität bereits geprüften ätherischen Öls von Salvia officinalis L..

Detektion:

Unter $UV_{254}$ war eine Fluoreszenzminderung erkennbar. Anschließend wurde die Platte für 5 Minuten bei 100 °C im Trockenschrank erhitzt. Die heiße Platte wurde dann mit einem frisch bereiteten Anisaldehyd-Schwefelsäure-Reagens (0,5 ml Anisaldehyd, 10 ml Eisessig, 5 ml konz. Schwefelsäure, 85 ml Methanol) besprüht und danach für weitere 10 Minuten im Trockenschrank erhitzt.

Ergebnisse:

Es trat eine Fluoreszenzminderung unter $UV_{254}$ bei den Chromatogrammen auf. Die Chromatogramme der Referenzlösungen zeigten nach dem Besprühen mit Anisaldehyd-Schwefelsäure-Reagens in der Mitte der Laufstrecke eine blauviolette Zone, die dem Cineol entsprach. Diese Zone war auch im Chromatogramm der Untersuchungslösung zu finden. Darüber war die schwach violette Zone des Thujon festzustellen. Im oberen Drittel befand sich eine leicht blaugrüne Bornylacetat-Zone. Im unteren Teil des Chromatogramms waren noch weitere Zonen sichtbar.

Dementsprechend konnte festgestellt werden, dass die Droge Salviae officinalis folium der Prüfung auf Identität entsprach.

Zwecks weiterer Charakterisierung der Droge wurde eine GC-Messung durchgeführt. Ziel war die Erstellung eines Fingerprints für Salviae officinalis folium. Ferner wurde die Größenordnung der einzelnen Bestandteile ermittelt.

Parameter für die GC-Analyse:

Gerät: Perkin-Elmer Auto System, FID

Säule:Kapillarsäule, 10 m x 0,25 mm i.D., Permabond OV-1-DF-0,50

Ofentemperatur: Programm 50 °C - 270 °C, 2,5 °C/Min. bei einer Initialzeit von 5 Minuten

Inj./Det. Temp.: 280 °C

Auswertung der GC-Analyse:

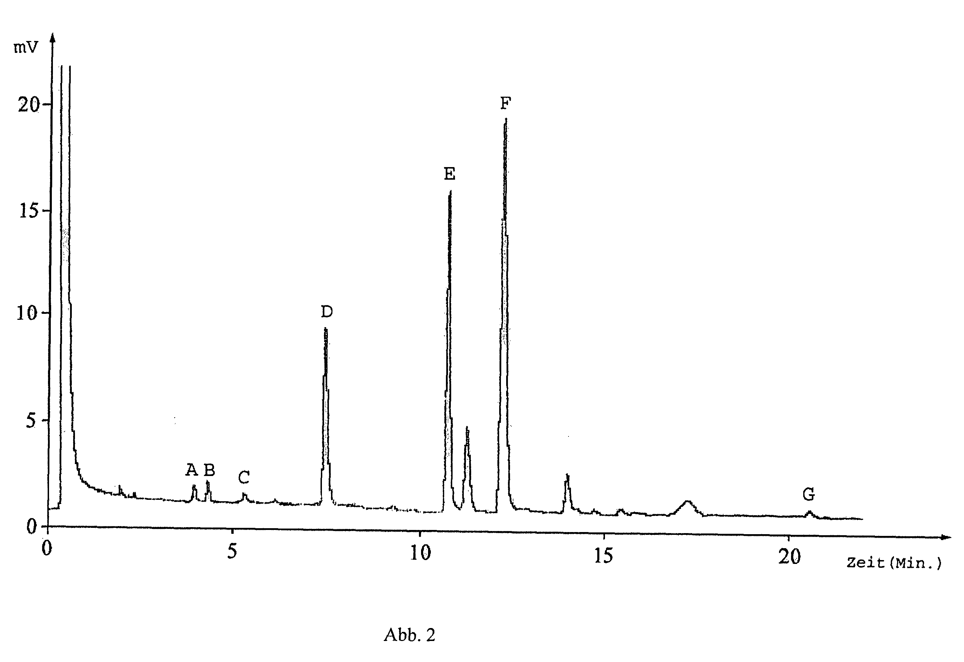

Das bei der GC erhaltene Chromatogramm (Abbildung 2) diente zur Erstellung eines Fingerprints. Bei Betrachtung des Chromatogramms kann man sowohl die für ein ätherisches Öl aus Salvia officinalis L. charakteristische Peakreihenfolge als auch die dazugehörigen Mengenverhältnisse herauslesen. Die Peaks von Cineol, Thujon und Campher sind deutlich zu erkennen, während $\alpha$-Pinen, Camphen, $\beta$-Pinen und Bornylacetat in geringer Menge auftreten. Auffallend ist der relativ hohe Gehalt an Campher. Die im Vergleich zum Thujon kleinere, aber dennoch verhältnismäßig große Peakfläche von Cineol lässt eine gewisse Verunreinigung der Droge Salviae officinalis folium mit Pflanzenmaterial von Salvia triloba L. fil. vermuten.

Da aber insgesamt die Menge an Thujon und Campher den Gehalt an Cineol deutlich übertraf, konnte die Identität der Droge Salviae officinalis folium bestätigt werden.

**B. Bestimmung des Inhaltsstoff-Gehalts**

**Beispiel 1B:** Calendula officinalis L.

Zur Bestimmung der Faradiolester diente ein an der Universität Wien, Institut für Pharmakognosie, entwickeltes Analyseverfahren. Es handelt sich um eine HPLC-Methode, die durch die Verwendung eines internen Standards eine sichere Quantifizierung der Faradiolester erlaubt. Die genaue Durchführung ist in Reznicek G., Zitterl-Eglseer K, 2003, Sci. Pharm., 71, 121 -128, nachzulesen.

Parameter für die Dünnschichtchromatographie:

Stat. Phase: KG 60 $F_{254}$ MERCK, Schichtdicke 0.25mm, 5 x 20 cm

Mob. Phase: n-Hexan-Ethylacetat (8+2)

Parameter für die HPLC-Analyse:

Pumpe: Perkin Elmer Series 200

Detektor: PE Series 200 UV/VIS Detector

Stat. Phase: LiChrospher RP-18, 5 μ, 4 x 250 mm

Vorsäule: LiChrospher RP-18, 5 μ, 4 x 40 mm

Mob. Phase: Gradient MeOH (p.A.-Qualität) - wässrige Trifluoressigsäure pH 4 (95+5) auf MeOH (p.A.-Qualität) - wässrige Trifluoressigsäure pH 4 (100+0) in 50 Minuten, dann MeOH (p.A.-Qualität)-wässrige Trifluoressigsäure pH 4 (100+0) 45 Minuten lang

Fluss: 1,5 ml/Minute

Detektion: UV 210 nm

Auswertung der HPLC-Analyse:

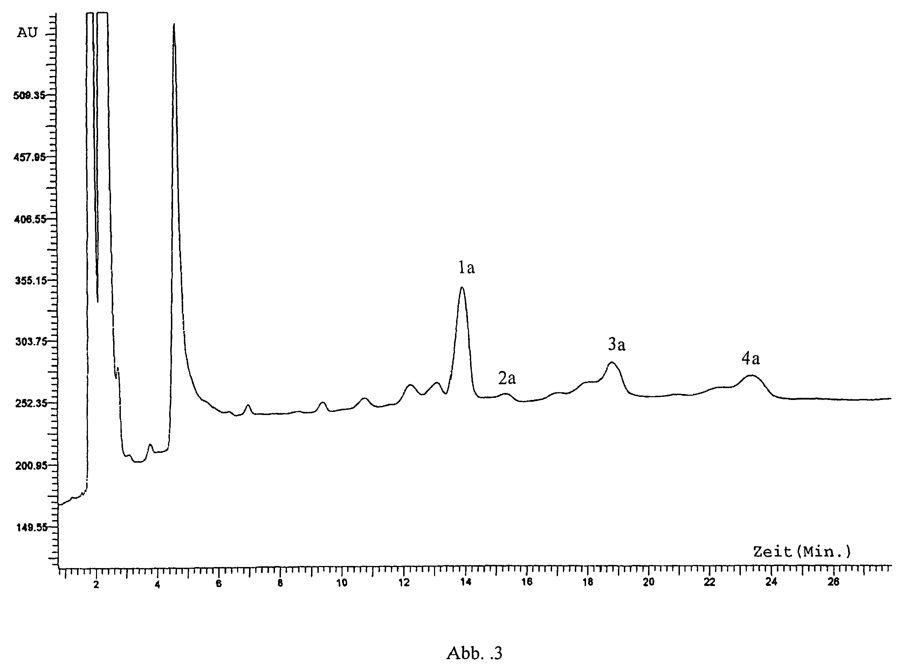

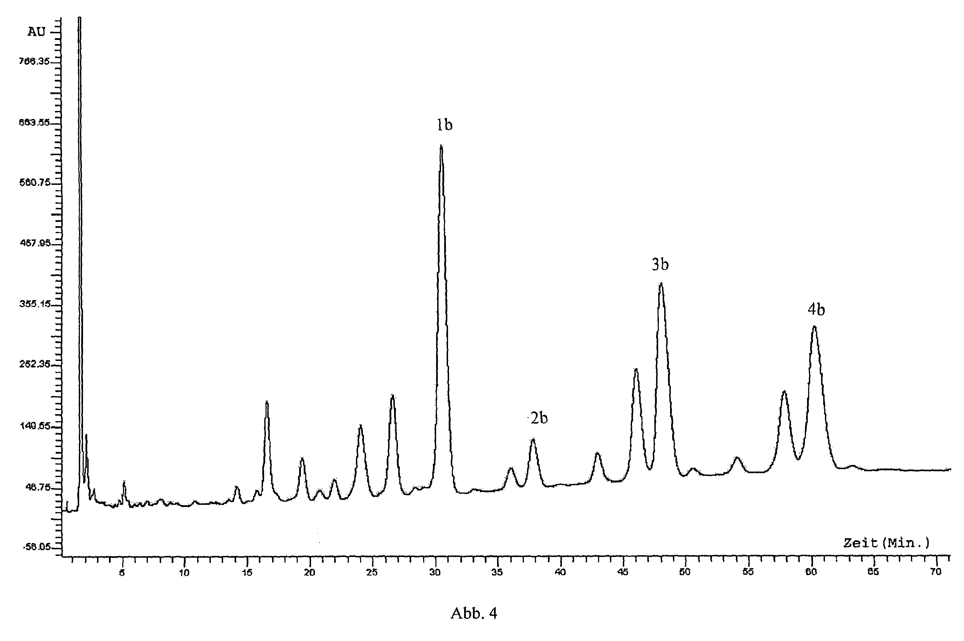

Durch Integration werden die Flächen des internen Standards und der Faradiolesterpeaks aus den Chromatogrammen (Abbildungen 3 und 4) ermittelt und zur Berechnung des Gehaltes der einzelnen Faradiolester in folgende Formel eingesetzt:

$$\% \, m_F = \frac{m_{St} \cdot A_F \cdot f_{St} \cdot 100}{A_{St} \cdot EW}$$

$m_F$   Menge des entsprechenden Faradiolesters in %
$m_{St}$   Menge zur Probe zugefügter Standard in mg
$A_F$   Peakfläche entsprechender Faradiolester
$A_{St}$   Peakfläche Standard
EW   Einwaage Droge in mg
$f_{St}$   Standardkorrekturfaktor:

$f_{st}$ (Faradiollaurat) = 1, 46

$f_{st}$ (Faradiolmyristat) = 1, 53

$f_{st}$ (Faradiolpalmitat) = 1, 60

Ergebnisse:

Wie aus den Tabellen 1 und 2 herauszulesen ist, besteht ein bedeutender Unterschied im Gehalt der Faradiolester zwischen "Calendula-Mischung" und "Calendula-Blüte". Diese Divergenz ergab sich zweifelsohne aus dem verschieden hohen Anteil an Zungenblüten in beiden Proben.
Bei der Auswertung der Ergebnisse der Proben von "Calendula-Mischung" aus der Ernte 2002 fiel auf, dass ebenso eine Differenz im Faradiolestergehalt zwischen zwei Original-Proben auftrat. Man kann diesen Unterschied wie folgt erklären: Es variierte auch die Zusammensetzung der einzelnen Proben untereinander, nämlich hinsichtlich eines abwechselnd hohen Anteils an Zungenblüten und Blütenstandboden. Daraus resultieren voneinander abweichende Faradiolestermengen.

Tabelle 1: Ergebnisse der quantitativen Auswertung der Faradiolester aus Calendulae flos nach HPLC-Analyse, Ernte 2001.

| | Original-Probe der Firma Waldland® ("Calendula-Mischung") | Probe der Firma Waldland®, die von allen laut EuAB nicht zulässigen Bestand-teilen befreit wurde ("Calendula-Blüte") |
|---|---|---|
| $m_{st}$ (mg) | 2,464 | 2,464 |
| $A_{st}$ | 45826227 | 19883039 |

(fortgesetzt)

| | Original-Probe der Firma Waldland® ("Calendula-Mischung") | Probe der Firma Waldland®, die von allen laut EuAB nicht zulässigen Bestand-teilen befreit wurde ("Calendula-Blüte") |
|---|---|---|
| Retentionszeit St (Min.) | 34,322 | 34,266 |
| Einwage Droge (mg) | 500,49 | 500,86 |
| Faradiollaurat: $A_F$/Retentionszeit (Min.) | 3262855 42,770 | 7129418 42,654 |
| Faradiolmyristat: $A_F$/Retentionszeit (Min.) | 25547790 53,539 | 37285453 53,384 |
| Faradiolpalmitat: $A_F$/Retentionszeit (Min.) | 21986101 66,483 | 32010716 66,384 |
| $m_F$ Faradiollaurat (in %) $m_F$ Faradiolmyristat (in %) $m_F$ Faradiolpalmitat (in %) | 0,0512 0,4199 0,3779 | 0,2575 1,4115 1,2672 |
| $m_F$ Gesamt (in %) | 0,8490 | 2,9362 |

Tabelle 2: Ergebnisse der quantitativen Auswertung der Faradiolester aus Calendulae flos nach HPLC-Analyse, Ernte 2002

| | Original-Probe der Firma Waldland® ("Calendula-Mischung") 1 | Original-Probe der Firma Waldland® ("Calendula-Mischung") 2 | Probe der Firma Waldland®, die von allen laut EuAB nicht zulässigen Bestandteilen befreit wurde ("Calendula-Blüte) |
|---|---|---|---|
| $m_{st}$ (mg) | 2,472 | 2,472 | 2,472 |
| $A_{st}$ | 32398417 | 33071495 | 52434554 |
| Retentionszeit St (Min.) | 30,63 | 30,47 | 30,54 |
| EW Droge (mg) | 502,10 | 504,00 | 500,86 |
| Faradiollaurat: $A_F$/Retentionszeit (Min.) | 2422342 38,10 | 3794193 37,67 | 6955468 37,80 |
| Faradiolmyristat: $A_F$/Retentionszeit (Min.) | 13198725 48,06 | 20637259 47,81 | 38120751 48,12 |
| Faradiolpalmitat: $A_F$/Retentionszeit (Min.) | 11641820 60,19 | 18211572 60,06 | 33800271 60,29 |
| $m_F$ Faradiollaurat (in %) $m_F$ Faradiolmyristat (in %) $m_F$ Faradiolpalmitat (in %) | 0,0537 0,3069 0,2831 | 0,0822 0,4683 0,4321 | 0,0956 0,5490 0,5090 |
| $m_F$ Gesamt (in %) | 0,6437 | 0,9826 | 1,1536 |

**Beispiel 2B:** Matricaria recutita L.

Der Gehalt des ätherischen Öls der Droge Matricariae flos wurde durch Wasserdampfdestillation ermittelt. Der Aufbau der dafür notwendigen speziellen Apparatur und die genaue Durchführung der Destillation sind im Europäischen Arzneimittelbuch (EuAB) von 1997 bei der "Gehaltsbestimmung des ätherischen Öls in Drogen" (2.8.12) nachzulesen. Unter Zuhilfenahme der entsprechenden Monographie wurde nach den im EuAB angegebenen Bedingungen gearbeitet: 30,0 g Droge wurden in einem 1000-ml-Rundkolben mit 300 ml Wasser (als Destillationsflüssigkeit) versetzt. Als Hilfsphase diente 0,5 ml Xylol. Für vier Stunden wurde mit einer Destillationsgeschwindigkeit von 3 bis 4 ml je Minute destilliert. Das durch Wasserdampfdestillation erhaltene ätherische Öl wurde dann gaschromatographisch analysiert.

Ergebnisse:

Die Auswertung der Gehaltsbestimmung des ätherischen Öls durch Wasserdampfdestillation ist der folgenden Tabelle zu entnehmen:

Tabelle 3: Gehalt an ätherischem Öl in der Droge Matricariae flos, Bestimmung durch Wasserdampfdestillation, Ernte 2001 und 2002.

| Einwaage Droge (in g) | Menge der Hilfsphase (Xylol, in ml) | Menge der Hilfsphase mit dem ätherischen Öl nach 4 h Destillation (in ml) | Menge äth. Öl (in %) |
|---|---|---|---|
| 30,70 | 0,505 | 0,570 | 0000,212 [1] |
| 30,34 | 0,500 | 0,570 | 0,231 [1] |
| 30,03 | 0,509 | 0,610 | 0,336 [2] |
| 30,08 | 0,500 | 0,592 | 0,36 [2] |
| [1] Kamillen-Droge von der Firma Waldland® , Ernte 2001 [2] Kamillen-Droge von der Firma Waldland® , Ernte 2002 | | | |

**Beispiel 3B:** Salvia officinalis L.

Der Gehalt des ätherischen Öls der Droge Salviae officinalis folium wurde durch Wasserdampfdestillation ermittelt. Der Aufbau der dafür notwendigen speziellen Apparatur und die genaue Durchführung der Destillation sind im EuAB 1997 bei der "Gehaltsbestimmung des ätherischen Öls in Drogen" (2.8.12) nachzulesen. Unter Zuhilfenahme der entsprechenden Monographie wurde nach den im EuAB angegebenen Bedingungen gearbeitet: 20,0 g Droge werden in einem 500-ml-Rundkolben mit 250 ml Wasser (als Destillationsflüssigkeit) versetzt. Als Hilfsphase dienen 0,5 ml Xylol. Für zwei Stunden wird mit einer Destillationsgeschwindigkeit von 3 bis 4 ml je Minute destilliert. Das durch Wasserdampfdestillation erhaltene ätherische Öl wurde gaschromatographisch analysiert.

Ergebnisse:

Die Auswertung der Gehaltsbestimmung des ätherischen Öls ist der folgenden Tabelle zu entnehmen:

Tabelle 4: Gehalt an ätherischem Öl in der Droge Salviae officinalis folium, Bestimmung durch Wasserdampfdestillation nach EuAB, Ernte 2001 und 2002

| Einwaage Droge (in g) | Menge der Hilfsphase (Xylol, in ml) | Menge der Hilfsphase mit dem ätherischen Öl nach 2 h Destillation (in ml) | Menge äth. Öl (in %) |
|---|---|---|---|
| 20,75 | 0,500 | 0,640 | 0,675 [1] |
| 20,41 | 0,501 | 0,640 | 0,681 [1] |
| 20,36 | 0,510 | 0,680 | 0,835 [2] |
| 20,45 | 0,498 | 0,663 | 0,807 [2] |
| 20,25 | 0,500 | 0,680 | 0,889 [2] |
| [1] Salbei-Droge von der Firma Waldland® , Ernte 2001 [2] Salbei-Droge von der Firma Waldland® , Ernte 2002 | | | |

Bestimmung des Gehaltes an Rosmarinsäure:

Der Gehalt an Rosmarinsäure wurde für die Salbei-Droge der Ernte 2001 mit Hilfe der Kapillarzonenelektrophorese bestimmt, deren genaue Durchführung, insbesondere Konditionierung und Reinigung der Kapillare sowie die Herstellung der für die Analyse notwendigen Pufferlösung, in Machart E., 2001, Dissertation, Universität Wien, 109 - 112 nachzulesen sind.

Für die Quantifizierung der Proben 2002 griff man auf ein anderes Hochleistungstrennverfahren zurück. Man verwendete für die Analysen die HPLC.

Untersuchungslösung:

Zwecks Aufbereitung der Probe wurden 0,5 g Droge mit 50 ml 20 %igem Methanol (p.A.-Qualität) versetzt und für 30 Minuten unter Rückfluss erhitzt. Nach dem Abkühlen wurde der Überstand abgetrennt und filtriert. Die verbliebene Droge wurde abermals mit 50 ml 20 %igem Methanol (p.A.-Qualität) versetzt und für 15 Minuten unter Rückfluss erhitzt. Anschließend wurde der abgekühlte Überstand erneut abgetrennt und filtriert. Danach wurde die Droge zum dritten Mal mit 50 ml 20 %igem Methanol (p.A.-Qualität) versetzt und für weitere 15 Minuten unter Rückfluss erhitzt. Die erhaltenen drei Teilfiltrate wurden gemeinsam zur Trockene unter Vakuum eingeengt. Der Rückstand wurde mit 5,00 ml 20 %igem Methanol (p.A.-Qualität) versetzt und für 5 Minuten am Ultraschallbad behandelt, um ein möglichst vollständiges Lösen der Rosmarinsäure zu gewährleisten. Anschließend wurde die Lösung für 5 Minuten bei 3500 U/Min. zentrifugiert.

Für die HPLC-Messung wurde der Überstand direkt entnommen und eine entsprechende Menge davon für die Analyse verwendet.

Für die Kapillarzonenelektrophorese-Messung wurden ungefähr 2,0 ml des entnommenen Überstandes zuvor noch durch einen AA-Filter (Porengröße von 1,0 $\mu$m) filtriert.

Parameter für die Kapillarzonenelektrophorese:

Gerät: SpectraPhoresis 1000 (SpectraPhysics Inc.)

Software: PC 1000, Version 3.0 unter IBM/OS2 von TSP (ThermoSeparationProducts)

Kapillare: fused silica Kapillare, 70 cm Länge x 50 $\mu$m Innendurchmesser

Spannung: 30 kV

Temperatur: 35 °C

Injektion: hydrodynamisch für 1 oder 3 Sek. an Anode

Detektion: UV-Detektion im Wellenlängenbereich 195- 360 nm

Puffer: 25 mM Natriumtetraborat pH 9,3 mit 20% Methanolzusatz

Injektionszeit: 3 Sek.

Laufzeit: 20 Min.

Detektionswellenlänge: 345 nm

Parameter für die HPLC-Analyse:

Pumpe: Perkin Elmer Series 200

Detektor: PE Series 200 UV/VIS Detector

Stat. Phase: LiChrospher RP-18,5 $\mu$, 4 x 250 mm

Vorsäule: LiChrospher RP-18,5 $\mu$, 4 x 40 mm

Mob. Phase: Gradient MeOH (p.A.-Qualität) - wässrige Trifluoressigsäure pH 2,6 (95+5) auf MeOH (p.A.-Qualität) - wässrige Trifluoressigsäure pH 2,6 (100+0) in 50 Minuten, dann MeOH (p.A.-Qualität)-wässrige Trifluoressigsäure pH 4 (100+0) 45 Minuten lang

Fluss: 1,5 ml/Minute

Detektion: UV 210 nm

Auswertung:

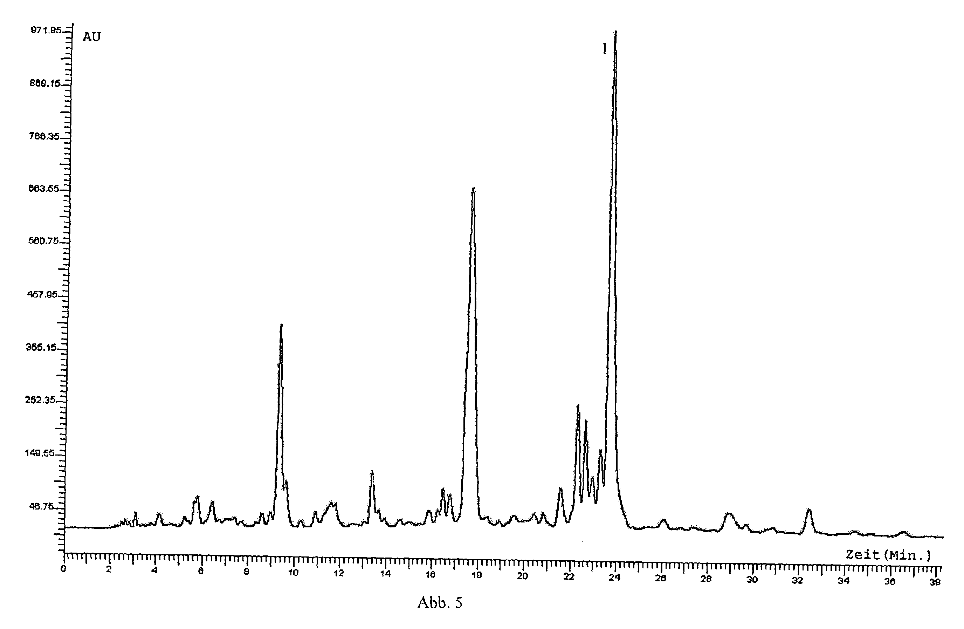

Um den Gehalt an Rosmarinsäure im Drogenauszug zu bestimmen, wurden Eichlösungen mit Rosmarinsäure in den Konzentrationen 0,5 bzw. 1,0 und 1,5 mg/ml hergestellt. Das Verhältnis von Einwaage zu Peakfläche der Rosmarinsäure aus den einzelnen Chromatogrammen (Abbildung 5) beziehungsweise Elektropherogrammen wurde gemittelt und für die Quantifizierung der Rosmarinsäure im Probenextrakt verwendet.

Die Peakflächen wurden durch Integration ermittelt und die Berechnung des Rosmarinsäuregehalts im Drogenauszug erfolgte nach folgender Formel:

$$\text{Gehalt in \% } = \frac{A_R \cdot V_{EW/FL} \cdot 100}{EW_{Droge}}$$

$A_R$...............Fläche der Rosmarinsäure in der Probe

$V_{EW/FL}$ ......... Verhältnis Einwaage Rosmarinsäure zu Peakfläche (Mittelwert aus den drei Eichlösungen)

$EW_{Droge}$..... Einwaage der Droge in mg

Ergebnisse:

Bei der Gehaltsbestimmung mittels Kapillarzonenelektrophorese erhielt man für die Rosmarinsäure Werte von 0,62 und 0,77 %. Die Ergebnisse der Ernte 2001 fielen damit noch in einen tolerablen Bereich.

Mit 0,8 und 0,9 % lag das Pflanzenmaterial der Ernte 2002 noch innerhalb eines akzeptablen Rahmens.

**C. Patientenstudie:**

Die Patientenstudie wurde mit insgesamt 40 Patienten durchgeführt, wobei für die Auswertung die Daten von 11 Patienten zur Verfügung standen.

Der bei der Auswertung verwendete Begriff "Patient" ist geschlechtsneutral zu verstehen.

Die Behandlung mit der erfindungsgemäßen Zusammensetzung bzw. mit dem entsprechenden Extrakt wurde an Patienten mit bereits vorhandenem Hand-Fuß-Syndrom durchgeführt.

Dabei wurde jeweils eine Mischung aus 10 g Matricariae flos und Salviae officinalis folium sowie 10 g Calendulae flos der Ernte 2001 bzw. 20 g der Ernte 2002 verwendet.

Es wurde nach dem oben beschriebenen Verfahren ein 3 Liter Badeaufguss hergestellt. Die Hand- und Fußbäder wurden zweimal täglich für jeweils 15 bis 20 Minuten durchgeführt. Die Patienten erhielten die abgewogene sowie sowohl auf ihre Identität und Quantität bestimmte Zusammensetzung und stellten den Extrakt und Hand- und Fußbäder selber her.

Die Behandlung mit Xeloda® erfolgte über vierzehn Tage, anschließend wurde mit einer siebentägigen Therapiepause fortgefahren. An dieses Schema angepasst, wurde den Patienten die Zusammensetzung für insgesamt 42 Tage - also für zwei vollständige Xeloda® -Zyklen plus zweimal einwöchiger Therapiepause - zur Verfügung gestellt. Die Anwendung der Zusammensetzung während der Xeloda® -Therapiepause sollte die durch das Absetzen des Chemotherapeutikums eingeleitete Abheilung der Symptome des Hand-Fuß-Syndroms unterstützen. Anschließend erfolgte ein dritter Xeloda® -Zyklus ohne eine Phytopharmakon-Therapie, um eine Veränderung des Hand-Fuß-Syndroms nach dem Absetzen der Phytopharmakon-Therapie zu dokumentieren. Die einzelnen Patienten konnten demnach über einen Zeitraum von insgesamt neun Wochen beobachtet werden.

Um die Ergebnisse der Behandlung mit der erfindungsgemäßen Zusammensetzung einheitlich auswerten zu können, wurden drei speziell an das Therapieschema der Phytopharmakon-Behandlung angepasste Fragebögen bzgl.

Schweregrad, Verlauf des Syndroms, sowie Anwendbarkeit, Akzeptanz und persönliches Empfinden ausgearbeitet. Jeweils der erste Teil der drei Fragebögen wurde vom behandelnden Arzt ausgefüllt. Damit sollte sichergestellt sein, dass die Beurteilung des entsprechenden Schweregrades der Hand-Fuß-Hautreaktion von einer fachkundigen Person durchgeführt wurde. Eine solche Vorgehensweise gewährleistet vergleichbare und reproduzierbare Ergebnisse. Der zweite Teil des jeweiligen Fragebogens diente der ausführlichen Beschreibung aller Symptome des Hand-Fuß-Syndroms durch den Patienten. Ebenso wurde auf die Darstellung der Lebensqualität und des gesamten Gesundheitszustandes sowohl vor als auch nach der Behandlung der erythematösen Hautveränderungen Wert gelegt. Die Ergebnisse der Fragebögen dienten - neben einer Bewertung der Wirksamkeit der Therapie - außerdem der Erkennung von etwaigen Problemen bei der Zubereitung und Anwendung der zweimal täglich angewendeten Hand- und Fußbäder.

Anhand einiger Fallbeispiele soll die Wirksamkeit der erfindungsgemäßen Zusammensetzung dargestellt werden.

**Patientenbeispiele 1 - 11:**

Alle 11 behandelten Patienten erhielten die oben beschriebene Zusammensetzung und wurden damit wie dargelegt behandelt.

Von den 11 behandelten Patienten verwendeten 6 parallel zur Behandlung mit der erfindungsgemäßen Zusammensetzung eine weitere Medikation, um dem Hand-Fuß-Syndrom entgegenzuwirken. Als zusätzlich zur Phytopharmakon-Lokaltherapie verwendete Produkte wurden unter anderem Betaisodona® Wundgel, Bepanthensalbe® , Hirschtalg® , Lanolin® , Vaseline® , Bag Balm® und am häufigsten Ringelblumensalbe genannt.

Compliance:

Mit Ausnahme von 2 Personen verwendeten alle Patienten die erfindungsgemäße Zusammensetzung jeweils für die Dauer des gesamten Xeloda® -Zyklus. Reisebedingte Unterbrechungen beziehungsweise ein Aussetzen der phytotherapeutischen Behandlung infolge eines stationären Krankenhausaufenthalts wurden als Gründe für ein Pausieren der Behandlung angeführt.

Ergebnisse:

Die Auswertung der Fragebögen ergab bei 7 Patienten eine Besserung des Hand-Fuß-Syndroms. Bei 2 Personen verbesserten sich einzelne Krankheitszeichen, jedoch ohne graduelle Änderung. Bei genauerer Charakterisierung wurde die Besserung von 8 Patienten als "deutlich" und von einer Person als "mäßig" bewertet. "Keine Veränderung der Symptomatik" wurde von einem Patienten angegeben. Eine Verschlechterung des Hand-Fuß-Syndroms war ebenfalls bei einem Behandelten zu verzeichnen.

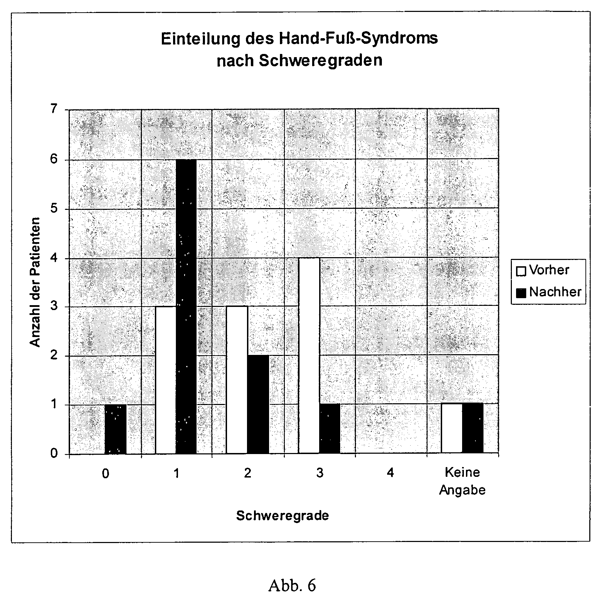

Wie aus Abbildung 6 ersichtlich, ergibt sich bei der Beurteilung des Hand-Fuß-Syndroms vor und nach der Behandlung mit der erfindungsgemäßen Zusammensetzung eine eindeutige Verschiebung in Richtung niedriger Schweregrade. Die Verdoppelung der Patientenanzahl mit einem Hand-Fuß-Syndrom vom Grad 1 ist auf ein vermindertes Auftreten von Grad 2 und 3 zurückzuführen. In einem Fall konnten - nach Anwendung der erfindungsgemäßen Zusammensetzung - keine auffälligen Hautreaktionen mehr festgestellt werden.

Keiner der 11 mit der erfindungsgemäßen Zusammensetzung behandelten Patienten konnte - abgesehen von den Symptomen des Hand-Fuß-Syndroms - ungewöhnliche Hautreaktionen beobachten.

Auf die Frage nach dem Auftreten der ersten Anzeichen einer Besserung des Hand-Fuß-Syndroms variierten die Angaben der Patienten von "gleich nach der ersten Behandlung" über "nach 2 Tagen" bis zu "nach einer Woche". Generell trat aber eine Besserung innerhalb der ersten 3 bis 4 Tage ein. Das Maximum der Besserung des Hand-Fuß-Syndroms durch eine Behandlung mit der erfindungsgemäßen Zusammensetzung konnte durchschnittlich nach einer Woche beobachtet werden. Am deutlichsten bildeten sich die Symptome der Hand-Fuß-Hautreaktion allerdings während der einwöchigen Xeloda® -Therapiepause zurück. Wie in Abbildung 7 zu sehen, kam es zu einer signifikanten Verminderung in der Einschränkung der täglichen Beschäftigungen.

Neben einer Reduktion der Einschränkungen im täglichen Leben beeinflusst ein positives Ansprechen der Symptome des Hand-Fuß-Syndroms auf die Behandlung die Beurteilung der momentanen Lebensqualität. So bestätigten 9 von 11 Patienten eine Verbesserung der Lebensqualität, wobei die Besserung von 5 Personen als "deutlich", von 3 Behandelten als "mäßig" und von einem Patienten als "minimal" beschrieben wurde. 1 Patient bemerkte keine Besserung der Lebensqualität.

Während 8 Personen von einer Besserung des Allgemeinzustandes berichteten, definierten 3 Patienten ihren Gesundheitszustand nach der Behandlung als unverändert.

9 Patienten bereitete die Zubereitung der Hand- und Fußbäder keine Schwierigkeiten, während 2 Personen die Aufbereitung als zu aufwendig empfanden.

Keiner der mit der Zusammensetzung behandelten Patienten musste im Laufe dieser Anwendungsbeobachtung aufgrund von Symptomen des Hand-Fuß-Syndroms als Folge der Chemotherapie mit Xeloda® stationär aufgenommen werden. Durch eine erfolgreiche Behandlung der entzündlichen Hautreaktionen mit dieser phytotherapeutischen Lokaltherapie konnte außerdem das (infolge starker entzündlicher Hautreaktionen indizierte) Ausweichen von einer oralen Xeloda® -Behandlung auf ein intravenöses Therapieschema verhindert werden.

**Patientenbeispiel 12:**

Unter der Anwendung der lokalen Phytopharmakon-Therapie entwickelte sich das Hand-Fuß-Syndroms eines Patienten von einem Grad 3 zu Grad 1. Aufgrund eines durch eingewachsene Zehnnägel bedingten chirurgischen Eingriffes an beiden großen Zehen, war eine Therapiemodulation notwendig, die eine Fortführung der beschriebenen Anwendung von Hand- und Fußbädern vorsah, mit Ausnahme beider großer Zehen, die während der Bäder über der Wasseroberfläche gehalten wurden. Daraufhin konnte beobachtet werden, dass es innerhalb weniger Tage an beiden großen Zehen zum Auftreten des Hand-Fuß-Syndroms (in Form von Blasen und intensiver Rötung) kam. Nach Entfernen des Verbandes von beiden großen Zehen war es wieder möglich, die Behandlung laut Anwendungsempfehlung durchzuführen, was in einer Rückbildung der Symptomatik des Hand-Fuß-Syndroms resultierte.

**Patentansprüche**

1. Zusammensetzung umfassend Calendula officinalis L. enthaltend Calendulae flos, Matricaria recutita L. enthaltend Matricariae flos und Salvia officinalis L. enthaltend Salviae officinalis folium.

2. Zusammensetzung nach Anspruch 1, **dadurch gekennzeichnet, dass** der Anteil an Calendulae flos 40 bis 60 Gew. % bezogen auf die Gesamtmenge Calendula officinalis L., der Anteil an Matricariae flos 40 bis 60 Gew. % bezogen auf die Gesamtmenge Matricaria recutita L. und der Anteil an Salviae officinalis folium 40 bis 60 Gew. % bezogen auf die Gesamtmenge Salvia officinalis L. beträgt.

3. Zusammensetzung nach Anspruch 1 oder 2, **dadurch gekennzeichnet, dass** Calendulae flos Chlorogensäure, Rutin, Hyperosid und Isorhamnetin-3-O-rutinosid umfasst.

4. Zusammensetzung nach einem der Ansprüche 1 bis 3, **dadurch gekennzeichnet, dass** die Calendulae flos mindestens 0,6 Gew. % Faradiolester umfasst bezogen auf die Gesamtmenge von Calendulae flos.

5. Zusammensetzung nach Anspruch 4, **dadurch gekennzeichnet, dass** die Faradiolester mindestens Faradiollaurat, Faradiolmyristat und Faradiolpalmitat umfassen.

6. Zusammensetzung nach einem der Ansprüche 1 bis 5, **dadurch gekennzeichnet, dass** Matricariae flos Herniarin, En-In-Dicycloether und α-Bisabolol umfasst.

7. Zusammensetzung nach einem der Ansprüche 1 bis 6, **dadurch gekennzeichnet, dass** Matricariae flos mindestens 0,2 Gew. % ätherisches Öl umfasst bezogen auf die Gesamtmenge von Matricariae flos.

8. Zusammensetzung nach einem der Ansprüche 1 bis 7 **dadurch gekennzeichnet, dass** Salviae officinalis folium Cineol, Thujon und Bornylacetat umfasst.

9. Zusammensetzung nach einem der Ansprüche 1 bis 8, **dadurch gekennzeichnet, dass** das ätherische Öl von Salviae officinalis folium mindestens Cineol, Thujon und Campher umfasst.

10. Zusammensetzung nach einem der Ansprüche 1 bis 9, **dadurch gekennzeichnet, dass** Salviae officinalis folium mindestens 0,6 Gew. % ätherisches Öl umfasst bezogen auf die Gesamtmenge von Salviae officinalis folium.

11. Zusammensetzung nach einem der Ansprüch 1 bis 10, **dadurch gekennzeichnet, dass** die Menge an Thujon und Campher jeweils den Cineol Anteil im ätherischen Öl von Salviae officinalis folium übertrifft.

12. Zusammensetzung nach Anspruch 11, **dadurch gekennzeichnet, dass** die Menge an Thujon und Campher zusammen mindestens 65 Gew.% bezogen auf die Gesamtmenge ätherisches Öl in Salviae officinalis folium beträgt.

**13.** Zusammensetzung nach einem der Ansprüche 1 bis 12, **dadurch gekennzeichnet, dass** der Rosmarinsäuregehalt von Salviae officinalis folium mindestens 0,6 Gew.% umfasst bezogen auf die Gesamtmenge von Salviae officinalis folium.

**14.** Extrakt umfassend eine Zusammensetzung nach einem der Ansprüche 1 bis 13.

**15.** Extrakt nach Anspruch 14, **dadurch gekennzeichnet, dass** der Extrakt ein Trockenextrakt ist.

**16.** Extrakt nach Anspruch 14, **dadurch gekennzeichnet, dass** der Extrakt ein flüssiges Extraktionsmittel umfasst.

**17.** Extrakt nach Anspruch 16, **dadurch gekennzeichnet, dass** der Extrakt ein flüssiges Extraktionsmittel, in einem Gewichtsverhältnis von Extrakt zu Extraktionsmittel von 1 zu 45 bis 1 zu 100 umfasst.

**18.** Extrakt nach Anspruch 16 oder 17, **dadurch gekennzeichnet, dass** das flüssige Extraktionsmittel Wasser ist.

**19.** Extrakt nach Anspruch 18, **dadurch gekennzeichnet, dass** der Extrakt 2 bis 4 Liter Wasser, 10 g Calendulae flos, 10 g Matricariae flos und 10 g Salviae officinalis folium umfasst.

**20.** Tinktur umfassend ein flüssiges Extraktionsmittel und eine Zusammensetzung nach einem der Ansprüche 1 bis 13.

**21.** Tinktur nach Anspruch 20, **dadurch gekennzeichnet, dass** die Tinktur die Zusammensetzung nach einem der Ansprüche 1 bis 13 zu 1 Anteil und zu 5 bis 10 Anteilen 30 bis 70 Vol.% igen wässrigen Ethanol umfasst.

**22.** Salbe, Creme, Emulsion oder Gel umfassend eine Zusammensetzung nach einem der Ansprüche 1 bis 13, eine hydrophile und/oder hydrophobe Komponente.

**23.** Verwendung der Zusammensetzung nach einem der Ansprüche 1 bis 13, eines Extraktes nach einem der Ansprüche 14 bis 19, einer Tinktur nach Anspruch 20 oder 21 oder einer Salbe, Creme, Emulsion oder Gel nach Anspruch 22 als Arzneimittel.

**24.** Verwendung einer Zusammensetzung nach einem der Ansprüche 1 bis 13, Extraktes nach einem der Ansprüche 14 bis 19, Tinktur nach Anspruch 20 oder 21, Salbe, Creme, Emulsion oder Gel nach Anspruch 22 zur Herstellung eines Arzneimittels zur Behandlung von erythematösen Hautveränderungen.

**25.** Verfahren zur Herstellung eines Extraktes nach einem der Ansprüche 14 bis 19, **dadurch gekennzeichnet, dass** die Zusammensetzung nach einem der Ansprüche 1 bis 13 zerkleinert sowie anschließend

- mit heißem Wasser übergossen wird,
- die Pflanzenrückstände abgetrennt werden
- und das so erhaltene wässrige Extrakt mit Wasser weiter verdünnt wird.

**26.** Verfahren nach Anspruch 25, **dadurch gekennzeichnet, dass** das Verhältnis Zusammensetzung nach einem der Ansprüche 1 bis 13 zu Wasser im erhaltenen wässrigen Extrakt 1 zu 45 bis 1 zu 50 beträgt und 40 bis 60 Anteile des so erhaltenen wässrigen Extraktes mit 60 bis 40 Anteilen Wasser weiter verdünnt werden.

**27.** Verfahren zur Herstellung eines Trockenextraktes nach Anspruch 15, **dadurch gekennzeichnet, dass** die Zusammensetzung nach einem der Ansprüche 1 bis 13 zerkleinert wird, mit einem geeigneten Extraktionsmittel extrahiert wird und anschließend der so erhaltene Extrakt bis zur Trockene eingedampft wird.

**28.** Verfahren zur Herstellung einer Tinktur nach Anspruch 20 oder 21 **dadurch gekennzeichnet, dass** die zerkleinerte Zusammensetzung nach einem der Ansprüche 1 bis 13 mit einem geeigneten Extraktionsmittel extrahiert wird und anschließend mit Ethanol versetzt wird.

**29.** Verfahren zur Herstellung einer Salbe, Creme, Emulsion oder Gel nach Anspruch 22 **dadurch gekennzeichnet, dass** die Zusammensetzung nach einem der Ansprüche 1 bis 13 in eine Salbengrundlage eingearbeitet wird.

**30.** Verfahren zur Herstellung einer Salbe, Creme, Emulsion oder Gel nach Anspruch 29, **dadurch gekennzeichnet, dass** zunächst Extrakte oder Tinkturen aus der Zusammensetzung nach einem der Ansprüche 1 bis 13 hergestellt werden und diese anschließend in hydrophile, hydrophobe/lipophile oder ambiphile Salben, Cremes, Emulsionen oder Gele eingearbeitet werden.

Abb. 1

Abb. 2

Abb. .3

Abb. 4

EP 1 707 213 A1

Abb. 5

Abb. 6

Abb. 7

**Europäisches Patentamt**

# EUROPÄISCHER RECHERCHENBERICHT

Nummer der Anmeldung

EP 05 00 5868

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | DATABASE WPI<br>Section Ch, Week 199936<br>Derwent Publications Ltd., London, GB;<br>Class B03, AN 1991-350856<br>XP002340042<br>& JP 02 929304 B2 (KOBAYASHI KOSE KK)<br>3. August 1999 (1999-08-03)<br>* Zusammenfassung *<br>----- | 1-30 | A61K35/78<br>A61P17/02 |
| Y | DATABASE WPI<br>Section Ch, Week 200019<br>Derwent Publications Ltd., London, GB;<br>Class B04, AN 2000-217932<br>XP002340043<br>& JP 2000 044481 A (SUNSTAR CHEM IND CO LTD) 15. Februar 2000 (2000-02-15)<br>* Zusammenfassung *<br>----- | 1-30 | |
| Y | DATABASE WPI<br>Section Ch, Week 200413<br>Derwent Publications Ltd., London, GB;<br>Class B04, AN 2004-126217<br>XP002340044<br>& JP 2003 277226 A (NOEVIR KK)<br>2. Oktober 2003 (2003-10-02)<br>* Zusammenfassung *<br>----- | 1-30 | |
| Y | US 4 163 049 A (AUBIN, MICHEL F)<br>31. Juli 1979 (1979-07-31)<br>*siehe Zusammenfassung, Spalte 1, Zeilen 5-17, Zeilen 32-38, Anspruch 1*<br>----- | 1-30 | RECHERCHIERTE SACHGEBIETE (Int.Cl.7)<br><br>A61K |
| Y | EP 0 424 534 A (KOOPERATIV "VIVATON"; DERYABIN, ALEXANDR MIKHAILOVICH)<br>2. Mai 1991 (1991-05-02)<br>*siehe Zusammenfassung, Seite 3, Zeilen 32-39, Anspruch 1*<br>----- | 1-30 | |

-/--

Der vorliegende Recherchenbericht wurde für alle Patentansprüche erstellt

| Recherchenort | Abschlußdatum der Recherche | Prüfer |
|---|---|---|
| München | 10. August 2005 | Stoltner, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

**Europäisches Patentamt**

# EUROPÄISCHER RECHERCHENBERICHT

Nummer der Anmeldung

EP 05 00 5868

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | DATABASE WPI<br>Section Ch, Week 200174<br>Derwent Publications Ltd., London, GB;<br>Class B04, AN 2001-642015<br>XP002340045<br>& JP 2001 226280 A (NOEVIR KK)<br>21. August 2001 (2001-08-21)<br>* Zusammenfassung *<br>----- | 1-30 | |
| A | WO 97/30719 A (BINDICS, FERENC)<br>28. August 1997 (1997-08-28)<br>*siehe Zusammenfassung, Seite 14, letzter<br>Absatz mit Seite 15, Absatz 1, Seite 18,<br>letzter Absatz, Anspruch 1*<br>----- | 1-30 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| München | 10. August 2005 | Stoltner, A |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.............................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                EP 05 00 5868

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-08-2005

| Im Recherchenbericht angeführtes Patentdokument | Datum der Veröffentlichung | Mitglied(er) der Patentfamilie | Datum der Veröffentlichung |
|---|---|---|---|
| JP 2929304 B2 | 03-08-1999 | JP 3236322 A | 22-10-1991 |
| JP 2000044481 A | 15-02-2000 | KEINE | |
| JP 2003277226 A | 02-10-2003 | KEINE | |
| US 4163049 A | 31-07-1979 | FR 2350103 A1 | 02-12-1977 |
| | | BE 854037 A1 | 28-10-1977 |
| | | DE 2720420 A1 | 01-12-1977 |
| | | GB 1517139 A | 12-07-1978 |
| | | IE 45752 B1 | 17-11-1982 |
| | | JP 53012411 A | 03-02-1978 |
| | | NL 7705033 A | 08-11-1977 |
| | | SE 7705268 A | 07-11-1977 |
| EP 0424534 A | 02-05-1991 | WO 9013305 A1 | 15-11-1990 |
| | | AU 632287 B2 | 24-12-1992 |
| | | AU 4183589 A | 29-11-1990 |
| | | DE 68916430 D1 | 28-07-1994 |
| | | DE 68916430 T2 | 13-10-1994 |
| | | EP 0424534 A1 | 02-05-1991 |
| | | JP 2703083 B2 | 26-01-1998 |
| | | JP 4501106 T | 27-02-1992 |
| | | NO 905605 A ,B, | 12-02-1991 |
| JP 2001226280 A | 21-08-2001 | KEINE | |
| WO 9730719 A | 28-08-1997 | AU 711191 B2 | 07-10-1999 |
| | | AU 1806697 A | 10-09-1997 |
| | | EP 0886523 A1 | 30-12-1998 |
| | | WO 9730719 A1 | 28-08-1997 |
| | | NO 983792 A | 15-10-1998 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LOPEZ A.M. et al.** *Cancer Chemother. Pharmacol.,* 1999, vol. 44, 303-306
- **COLEMAN et al.** berichten auf der Tagung der American Society of Clinical Oncology. *ASCO's,* 2001
- **HÄNSEL R.** Pharmakognosie - Phytopharmazie. Springer Verlag, 1999, vol. 7, 524-526699-704740-743
- Kamillosan® im Spiegel der Literatur. 1991, 24-27
- **BARICEVIC C.H.** *Zeitschrift für Phytotherapie,* 1991, vol. 12, 61-69
- **HÄNSEL R. et al.** Hagers Handbuch der Pharmazeutischen Praxis. Springer Verlag, 1992, vol. 4, 817-831597-615
- HAGERS HANDBUCH DER PHARMAZEUTISCHEN PRAXIS. SPRINGER VERLAG, 1994, vol. 6, 538-574
- **WICHTL M.** Teedrogen und Phytopharmaka: Ein Handbuch für die Praxis wissenschaftlicher Grundlage. Wiss. Verl.-Ges, 2002, 100-103. 369373, 538-574
- **HÄNSEL R. et al.** Pharmakognosie - Phytopharmazie. Springer Verlag, 1999, 524-526699-704740-743
- **HÄNSEL R. ; STICHER O. ; STEINEGGER E.** Pharmakognosie - Phytopharmazie. Springer Verlag, 1999, 524-526699-704740-743
- **HÄNSEL R. ; KELLER K. ; RIMPLER H.** Hagers Handbuch der Pharmazeutischen Praxis. Springer Verlag, 1992, vol. 4, 817-831597-615

- **WICHTL M.** Teedrogen und Phytopharmaka: Ein Handbuch für die Praxis wissenschaftlicher Grundlage. Wiss. Verl.-Ges, 2002, 100-103. 369373, 538-542
- **HÄNSEL R.** Pharmakognosie - Phytopharmazie. Springer Verlag, 1999, 524-526699-704740-743
- **GRUNDWERK.** Ph. Eur. 2002, 710-711
- Ph. Eu. vol. 3, 3765
- **GRUNDWERK.** Ph. Eur. 2002, 711-712
- Ph. Eu. 3767
- **GRUNDWERK.** Ph. Eur. 2002, 712
- Pharmacopoea Austriaca. Verlag Österreich, 1990, vol. II
- **GRUNDWERK.** Ph. Eur. 2002, 739-740
- Ph. Eu. 3766-3767
- ÖAB (Pharmacopoea Austriaca. Verlag Österreich, 1990, vol. II
- **GRUNDWERK.** Ph. Eur. 2002, 752-753
- Ph. Eu. 3776-3777
- **GRUNDWERK.** Ph. Eur. 2002, 753
- Ph. Eu. 3777
- **WAGNER H. ; BLADT S.** A Thin Layer Chromatography Atlas. Springer-Verlag, 1996, 216-217
- *EuAB,* 1997, vol. 129 - 13, 1161-1162
- **REZNICEK G. ; ZITTERL-EGLSEER K.** *Sci. Pharm.,* 2003, vol. 71, 121-128
- **MACHART E.** Dissertation. Universität Wien, 2001, 109-112